(19) Europäisches Patentamt
European Patent Office
Office européen des brevets

(11) EP 4 293 117 A1

(12) EUROPEAN PATENT APPLICATION
published in accordance with Art. 153(4) EPC

(43) Date of publication:
**20.12.2023 Bulletin 2023/51**

(21) Application number: **21925597.3**

(22) Date of filing: **10.02.2021**

(51) International Patent Classification (IPC):
***C12N 15/55*** *(2006.01)* ***C12Q 1/26*** *(2006.01)*
***C12Q 1/34*** *(2006.01)* ***C12N 9/06*** *(2006.01)*
***C12N 9/14*** *(2006.01)* ***G01N 33/50*** *(2006.01)*

(52) Cooperative Patent Classification (CPC):
**C12N 9/0004; C12Q 1/26; C12Q 1/34; G01N 33/50;**
C12N 9/14

(86) International application number:
**PCT/JP2021/004869**

(87) International publication number:
**WO 2022/172343 (18.08.2022 Gazette 2022/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Oriental Yeast Co., Ltd.**
**Tokyo 174-8505 (JP)**

(72) Inventors:
- **ICHIMARU, Kenta**
**Nagahama-shi, Shiga 526-0804 (JP)**
- **MATSUKAWA, Hirokazu**
**Nagahama-shi, Shiga 526-0804 (JP)**
- **MIYACHI, Mayu**
**Nagahama-shi, Shiga 526-0804 (JP)**
- **TAKETANI, Yukiko**
**Nagahama-shi, Shiga 526-0804 (JP)**
- **IKENO, Chizuka**
**Nagahama-shi, Shiga 526-0804 (JP)**
- **SUNAHARA, Yoshiko**
**Nagahama-shi, Shiga 526-0804 (JP)**

(74) Representative: **Hoffmann Eitle**
**Patent- und Rechtsanwälte PartmbB**
**Arabellastraße 30**
**81925 München (DE)**

Remarks:
The complete document including Reference Table(s) and the Sequence Listing(s) can be downloaded from the EPO website

# (54) URICASE ACTIVATOR AND URIC ACID MEASUREMENT REAGENT

(57) Provided are a uricase activator and a uricase activation method which are capable of highly activating uricase. Also provided are a uric acid measurement reagent and a uric acid measurement method which have a wide measurable concentration range. Further provided are a uricase activator comprising hydroxyisourate hydrolase, and a uric acid measurement reagent for use in measuring a uric acid concentration in a sample collected from a living body, comprising uricase and hydroxyisourate.

EP 4 293 117 A1

## Description

Technical Field

The present invention relates to a uricase activator and a uric acid measurement reagent comprising hydroxyisourate hydrolase, and a uricase activation method and a uric acid measurement method using hydroxyisourate hydrolase.

Background Art

Uric acid is a main end product of purine metabolism in humans and is produced in a body and excreted through urine or feces. Uric acid in blood is maintained at a constant level by achieving a balance between the production and excretion of uric acid from purine bodies. However, due to a diet, a genetic factor, an environmental factor, or the like, the balance may be lost so that a uric acid concentration in a body (in blood or in urine) elevates, developing hyperuricemia. Hyperuricemia is known not only to be responsible for gout but to cause many complications such as renal damage and vascular damage. For early detection and treatment thereof, it is important to measure a uric acid concentration in blood or in urine, particularly, in blood. A serum uric acid level is an examination item that is widely measured in comprehensive medical examination or the like.

Patent Literature 1 discloses PEGylated uricase (urate oxidase) effective for the treatment of hyperuricemia. Patent Literatures 2 and 3 disclose a method for measuring uric acid by using uricase. Patent Literature 4 has reported a plurality of uricases that can be used in uric acid measurement. The uric acid measurement methods described in Patent Literatures 2 to 4 involve allowing uricase to act on uric acid to generate 5-hydroxyisourate and hydrogen peroxide, detecting or measuring the generated hydrogen peroxide using various reagents or approaches, and thereby measuring the concentration of the uric acid. In this respect, the 5-hydroxyisourate generated together with hydrogen peroxide is an unstable substance and is usually nonenzymatically decarboxylated and converted into allantoin.

Meanwhile, Patent Literature 5 and Non Patent Literature 1 state that the degradation of uric acid is catalyzed at three stages not only by uricase but by 5-hydroxyisourate hydrolase (HiUH) which catalyzes the hydrolysis of 5-hydroxyisourate (HiU) produced from the uric acid into 2-oxo-4-hydroxy-4-carboxy-5-ureidoimidazoline (OHCU), and 2-oxo-4-hydroxy-4-carboxy-5-ureidoimidazoline decarboxylase (OHCUD) which catalyzes the conversion of OHCU into allantoin through decarboxylation. Reported HiUH is derived from vertebrates, plants, or bacteria (Patent Literature 5 and Non Patent Literatures 1 to 9).

Prior Art

Patent Literature

Patent Literature 1: JP Patent Publication No. 2002-522399 A (2002)
Patent Literature 2: International Publication No. WO 2006/030866
Patent Literature 3: JP Patent Publication No. 06-070798 A (1994)
Patent Literature 4: JP Patent Publication No. 06-038766 A (1994)
Patent Literature 5: International Publication No. WO 2007/052326


Non Patent Literature

Non Patent Literature 1: Y. Lee et al., FEBS Letters 579 (2005) 4769-4774
Non Patent Literature 2: K. Yamauchi and K. Kasai, J. Mol. Evol., 86 (2018) 457-469
Non Patent Literature 3: G. Zanotti, et al., J. Mol. Biol., 363 (2006) 1-9
Non Patent Literature 4: A. Raychaudhuri and P. A. Tipton, Plant Physiol,. 130 (2002) 2061-2068
Non Patent Literature 5: J. Pessoa et al., BMC Plant Biology, 20 (2010) 30
Non Patent Literature 6: E. Lundberg et al., FEBS Journal, 276 (2009) 1999-2011
Non Patent Literature 7: C. Matiollo et al., BBRC, 387 (2009) 712-716
Non Patent Literature 8: J. B. French and S. E. Ealick, Acta. Cryst., D67 (2011) 671-677
Non Patent Literature 9: S. He, et al., Appl. Environ. Microbiol., 85 (19) e01107-19 (2019)

## Summary of Invention

### Object to be Achieved by the Present Invention

Although the function of 5-hydroxyisourate hydrolase (HiUH) in uric acid metabolism is known, enzymological characteristics of HiUH are difficult to exhaustively elucidate because 5-hydroxyisourate (HiU) serving as a substrate is unstable in an aqueous solution. Hence, application thereof to the measurement of the level of uric acid comprised in blood or in urine has not been practiced.

An object of the present invention is to provide a uricase activator and a uricase activation method which are capable of highly activating uricase. Another object of the invention is to provide a uric acid measurement reagent and a uric acid measurement method which have a wide measurable concentration range.

### Means to Achieve the Object

The present inventors have conducted diligent studies and consequently found that the catalysis of conversion of uric acid into 5-hydroxyisourate (HiU) by uricase is more activated in the presence of HiUH and thus permits the conversion of a higher concentration of uric acid. The inventors have further found that uricase activity exhibits high stability in the presence of HiUH. The invention has been completed on the basis of these findings.

Specifically, the invention provides the following.

(1) A uricase activator comprising hydroxyisourate hydrolase.

(2) The activator according to (1), wherein the hydroxyisourate hydrolase has at least one amino acid sequence selected from the following amino acid sequences (i) and (ii), and has catalytic activity on the hydrolysis of 5-hydroxyisourate into 2-oxo-4-hydroxy-4-carboxy-5-ureidoimidazoline:

(i) an amino acid sequence represented by the following formula (I) or (II):

[K/H]-[I/V]-L-[D/N]-x-x-x-G-x-P-[A/G]-x-x-[L/I/V/M]-x-[I/V] ... (I)

[Y/W/F]-[T/H]-[I/V/T]-[A/P]-x-x-[L/I/V/M]-[S/T/A]-[P/Q]-[F/Y/W/G]-[G/S]-[F/Y]-[Q/S/T] ... (II)

wherein x represents any amino acid, and

(ii) an amino acid sequence which is different from any one of amino acid sequence (i) with the substitution, deletion, or addition of one to three amino acids.

(3) The activator according to (1) or (2), wherein the uricase activator is used for activating uricase by allowing the uricase activator to be present together with the uricase, and a content of the hydroxyurate hydrolase is 0.005 to 1.5 times of an amount of the uricase present together in terms of a weight ratio.

(4) The activator according to any of (1) to (3), wherein the uricase activator increases the activity of uricase to 1.2 or more times.

(5) The activator according to any of (1) to (4), wherein the hydroxyisourate hydrolase is a thermostable enzyme.

(6) The activator according to any of (1) to (5), wherein specific activity of the hydroxyisourate hydrolase is 100 U/mg or more.

(7) The activator according to any of (1) to (6), wherein the hydroxyisourate hydrolase is derived from the genus *Bacillus,* the genus *Herbaspirillum,* or the genus *Deinococcus.*

(8) The activator according to any of (1) to (6), wherein the hydroxyisourate hydrolase has any of the following features (a) to (c):

(a) having the amino acid sequence of SEQ ID NO: 10;

(b) having an amino acid sequence having 70% or higher identity to the amino acid sequence of SEQ ID NO: 10, and having catalytic activity on the hydrolysis of 5-hydroxyisourate into 2-oxo-4-hydroxy-4-carboxy-5-ureidoimidazoline; and

(c) having an amino acid sequence which is different from the amino acid sequence of SEQ ID NO: 10 with the deletion, substitution, or addition of one or several amino acids, and having catalytic activity on the hydrolysis of 5-hydroxyisourate into 2-oxo-4-hydroxy-4-carboxy-5-ureidoimidazoline.

(9) The activator according to any of (1) to 8, wherein the hydroxyisourate hydrolase is derived from *Deinococcus*

*radiodurans.*

(10) A uricase activation method comprising the step of allowing uricase and a uricase activator according to any of (1) to (9) to be present together.

(11) The method according to (10), wherein the step is the step of allowing the uricase and the uricase activator to be present together at a weight ratio of from 1:0.005 to 1:1.5.

(12) A uric acid measurement reagent for use in measuring a uric acid concentration in a sample collected from a living body, comprising uricase and hydroxyisourate hydrolase.

(13) The reagent according to (12), wherein the hydroxyisourate hydrolase has at least one amino acid sequence selected from the following amino acid sequences (i) and (ii), and has catalytic activity on the hydrolysis of 5-hydroxyisourate into 2-oxo-4-hydroxy-4-carboxy-5-ureidoimidazoline:

(i) an amino acid sequence represented by the following formula (I) or (II):

[K/H]-[I/V]-L-[D/N]-x-x-x-G-x-P-[A/G]-x-x-[L/I/V/M]-x-[I/V] ... (I)

[Y/W/F]-[T/H]-[I/V/T]-[A/P]-x-x-[L/I/V/M]-[S/T/A]-[P/Q]-[F/Y/W/G]-[G/S]-[F/Y]-[Q/S/T] ... (II)

wherein x represents any amino acid, and

(ii) an amino acid sequence which is different from any one amino acid sequence (i) with the substitution, deletion, or addition of one to three amino acids.

(14) The reagent according to (12) or (13), wherein a weight concentration ratio of the uricase to the hydroxyurate hydrolase is from 1:0.005 to 1:1.5.

(15) The reagent according to any of (12) to (14), wherein the hydroxyisourate hydrolase is a thermostable enzyme.

(16) The reagent according to any of (12) to (15), wherein specific activity of the hydroxyisourate hydrolase is 100 U/mg or more.

(17) The reagent according to any of (12) to (16), wherein the hydroxyisourate hydrolase is derived from the genus *Bacillus,* the genus *Herbaspirillum,* or the genus *Deinococcus.*

(18) The reagent according to any of (12) to (17), wherein the hydroxyisourate hydrolase has any of the following features (a) to (c):

(a) having the amino acid sequence of SEQ ID NO: 10;

(b) having an amino acid sequence having 70% or higher identity to the amino acid sequence of SEQ ID NO: 10, and having catalytic activity on the hydrolysis of 5-hydroxyisourate into 2-oxo-4-hydroxy-4-carboxy-5-ureidoimidazoline; and

(c) having an amino acid sequence which is different from the amino acid sequence of SEQ ID NO: 10 with the deletion, substitution, or addition of one or several amino acids, and having catalytic activity on the hydrolysis of 5-hydroxyisourate into 2-oxo-4-hydroxy-4-carboxy-5-ureidoimidazoline.

(19) The t reagent according to any of (12) to (18), wherein the hydroxyisourate hydrolase is derived from *Deinococcus radiodurans.*

(20) A uric acid measurement method comprising the step of mixing and reacting a uric acid measurement reagent according to any of (12) to (19) with a sample collected from a living body.

Advantageous Effects of Invention

The present invention can provide a uricase activator and a uricase activation method which are capable of highly activating uricase. Also, the invention can provide a uric acid measurement reagent and a uric acid measurement method which have a wide measurable concentration range.

Brief Description of Drawings

[Figure 1] Figure 1 is a reaction formula of the degradation of uric acid by the uric acid measurement reagent of the present invention.

[Figure 2] Figure 2 is a graph showing the uricase activity of a uricase reaction test solution comprising HiUH derived

from DR, BS, or HS. When the amount of change in absorbance caused by uricase under HiUH-free conditions was defined as 100%, a relative value (%) of the amount of change of each test solution was calculated. Figure 2A shows the influence of HiUH on the activity of uricase derived from the genus *Bacillus.* Figure 2B shows the influence of HiUH on the activity of uricase derived from a yeast.

[Figure 3] Figure 3 is a graph in which a theoretical concentration value of uric acid (abscissa) is plotted against a measured concentration value measured with a measurement reagent comprising HiUH or comprising no HiUH (ordinate) as to each solution of uric acid dilution series.

[Figure 4] Figure 4 is a graph in which a calculated ratio (%) of a measured concentration value measured with a measurement reagent comprising HiUH or comprising no HiUH to a theoretical concentration value of uric acid is plotted against the theoretical value as to each solution of uric acid dilution series.

[Figure 5] Figure 5 is a graph in which a theoretical concentration value of uric acid (abscissa) is plotted against a measured value of change in absorbance measured using a reagent after implementation of each acceleration test (ordinate) as to each solution of uric acid dilution series. Figure 5A shows a measured value of a control. Figure 5B shows a measured value obtained using a reagent comprising HiUH at twice the active concentration of uricase. Figure 5C shows a measured value obtained using a reagent comprising HiUH at three times the active concentration of uricase.

[Figure 6] Figure 6 is a molecular phylogenetic tree constructed on the basis of identity to the sequence of DR HiUH gene as to HiUH genes derived from various organisms.

## Description of Embodiments

In the present specification, "activity" of an enzyme refers to a value determined under a boric acid-free condition of pH 7.0 unless otherwise specified. "Specific activity" refers to activity per mg of an enzyme protein. One unit (U) of enzyme activity refers to the amount of an enzyme that catalyzes 1 μmol of a substrate for 1 minute under a condition of 25°C unless otherwise specified.

In the specification, "thermostability" or "heat resistance" of an enzyme is determined on the basis of change in enzyme activity between before and after heat treatment when the heat treatment is performed at 60°C for 30 minutes, unless otherwise specified. In the specification, "% (percent)" refers to % by weight if indicating a concentration, unless otherwise specified.

### <Uricase activator>

The uricase activator of the invention comprises hydroxyisourate hydrolase. The uricase activator of the invention has an effect of highly activating uricase by comprising hydroxyisourate hydrolase.

In the specification, "uricase" is an enzyme for uric acid as a substrate and is an enzyme that catalyzes reaction of degrading uric acid into 5-hydroxyisourate (HiU) and hydrogen peroxide. Any uricase known in the art can be used as long as the enzyme has the catalytic activity described above. Uricase derived from any organism such as a vertebrate including a mammal, bird, and fish (however, naturally occurring uricase is known to be absent in primates), an invertebrate, a plant, a fungus (e.g., a yeast), and a bacterium may be used. For example, recombinant uricase produced by functionally integrating uricase gene derived from a bacterium or a fungus in a host (e.g., *E. coli*) may be used. In this case, the bacterium of origin or the fungus of origin is not particularly limited. For example, uricase derived from the genus *Bacillus,* a yeast, or the genus *Arthrobacter* may be used. Particularly, uricase derived from the genus *Bacillus* is preferred because of being excellent in stability.

In the specification, the uricase may be an enzyme alone or may be an enzyme conjugated with a polymer such as polyethylene glycol (PEG). Usually, the former is often used in a uric acid measurement reagent, and the latter may be used in a therapeutic drug for hyperuricemia or the like. Any form of uricase is applicable to the uricase activator of the invention.

In the specification, "5-hydroxyisourate hydrolase (HiUH)" is a hydrolytic enzyme that catalyzes reaction of hydrolyzing HiU into 2-oxo-4-hydroxy-4-carboxy-5-ureidoimidazoline (OHCU). The HiUH for use in the uricase activator of the invention is not particularly limited by its organism of origin or structure as long as the enzyme has the activity described above. HiUH derived from any organism such as a vertebrate including a mammal, bird, and fish, an invertebrate, a plant, a fungus (e.g., a yeast), and a bacterium may be used. For example, the amino acid sequence of HiUH from a mouse (the amino acid sequence of SEQ ID NO: 1; see Patent Literature 5), *Branchiostoma japonicum* (SEQ ID NO: 2; see Non Patent Literature 2), *Danio rerio* (SEQ ID NO: 3; see Non Patent Literature 3), or *Oncorhynchus kisutch* (SEQ ID NO: 4; see Non Patent Literature 2) has been reported for animals. For example, the amino acid sequence of HiUH from *Glycine max* (SEQ ID NO: 5; see Non Patent Literature 4) or *Arabidopsis thaliana* (SEQ ID NO: 6; see Non Patent Literature 5) has been reported for plants. For example, the amino acid sequence of HiUH from *Escherichia coli* (SEQ ID NO: 7; see Non Patent Literature 6), *Bacillus subtilis* (SEQ ID NO: 8; see Non Patent Literature 1), a bacterium

of the genus *Herbaspirillum* (SEQ ID NO: 9; see Non Patent Literature 7), *Deinococcus radiodurans* (SEQ ID NO: 10, UniProtKB Accession No. Q9RV69), *Klebsiella pneumoniae* (SEQ ID NO: 11; see Non Patent Literature 8), or *Salmonella enterica* (SEQ ID NO: 12; see Non Patent Literature 9) has been reported for bacteria.

HiUH is known to have a structure similar to that of transthyretin (TTR). TTR is also present in human blood and is known to work as a transport carrier for a thyroid hormone thyroxin or vitamin A. Human TTR has been reported as a protein having the amino acid sequence represented by SEQ ID NO: 13 (see Non Patent Literature 6). A group of proteins structurally similar to TTR is also called transthyretin-related protein (TRP) and is widely present in non-human animals, plants, bacteria, fungi, and the like. TTR and TRP are known to have highly conserved motif sequences between different species in their amino acid sequences. It is further known that some proteins of TRP having the motifs have activity of hydrolyzing HiU, i.e., such proteins are HiUH. In the present specification, the HiUH includes, but is not limited to, general proteins having HiU-hydrolyzing activity which are included in TRP, regardless of a species of origin.

Exemplary amino acid sequences comprised in known HiUH and human TTR are shown in Table 1, though the HiUH of the invention is not limited to those comprising these amino acid sequences.

[Table 1]

| Organism species | Amino acid sequence | SEQ ID NO |
|---|---|---|
| *Mus musculus* | MATESSPLTTHVLDTASGLPAQGLCLRLSRLEAPCQ QWMELRTSYTNLDGRCPGLLTPSQIKPGTYKLFFDT ERYWKERGQESFYPYVEVVFTITKETQKFHVPLLLS PWSYTTYRGS | 1 |
| *Branchiostoma japonicum* | MGCPAEIYVSSDHQKKKLVVTRNNDNHNHESPAISP SPLAMSANRTSPITTHILDTSLGRPAADVPIKLYRR AERIGQEWSQVSSGQTNSDGRCNGLLNSLEAGVYKI TFETATYFNKNGIRQYFYPYVDIVFEIQDPIQHYHV PLLLNPFGYSTYRGS | 2 |
| *Danio rerio* | MAATLLSPLSTHVLNIAQGVPGANMTIVLHRLDPVS SAWNILTTGITNDDGRCPGLITKENFIAGVYKMRFE TGKYWDALGETCFYPYVEIVFTITNTSQHYHVPLLL SRFSYSTYRGS | 3 |
| *Oncorhynchus kisutch* | MTLKAAHMSTSRLQHIKDHILDEYTCAEMAAPYSPL TTHVLNTGMGVPGAHMALSLHRMDPSTSLWNLLTTG TTNDDGRCPGLITRETFTPAVYKIRFETGQYWGSLG ETSFYPYVEIVFTITDHSQKFHVPLLCSRFSYTTYR GS | 4 |
| *Glycine max* | ADNYSRDDFPLDFVFGSGTSAYQVEGAANKDGRTPS IWDTFAYAGYAHGENGDVACDGYHKYKEDVQLMLET GLDAYRFSISWSRLLPNGRGPVNPKGLQYSNNLINE LISNGIQPHATLYNFDLPQVLEDEYGGWISRDIIRD FTYYAEVEFREFGDRVLYWTTVNEPNVFALGGYDQG NSPPRRCSPPFCATNDTMGNSTYEPYLAVHHILLSH SSAARLYWRKYRDKQHGFVGISIYTFGIFPQTNTEK DRVASQRARDFFVGWIMEPLQYGDYPISMKTNAGER IPAFTNHESKQVKGSFDFIGVIHYTNLNVSDNSDAL KNQLRDFTADMAANIFGEDLFSNEEYLITPWGLRQE | 5 |

(continued)

| Organism species | Amino acid sequence | SEQ ID NO |
|---|---|---|
| | LNKFKLLYGNPPIFIHENGQRTASNSSLQDVDKGEI LHGYIGSVLDALRDASNIKGYFRMAFPGFVRVARWI QVSFGLYYVDRDDPQLKKIPKLFCKNGTTGFLKGRR TSILDLFELEQDPITCSKSPIIFSKISKWVLASLLF LIQHKIKFMWREPLPGQIPLKLVMF | |
| *Arabidopsis thaliana* | MAMEIGEDEWKVCCGSSEFAKQMSTSGPLTSQEAIY TARDIWFNQVNVTDWLEAFSAHPQIGNTPSPSINSD FARRSVSEQSTAFATTSASALQELAEWNVLYKKKFG FIFIICASGRTHAEMLHALKERYENRPIVELEIAAM EQMKITELRMAKLFSDKAKVISETDSSSSPVSTKPQ DRLRIIGGHLNVAAEAKAPKRSRPPITTHVLDVSRG APAAGVEVHLEVWSGTTGPSFVHGGGGVWSSVGTSA TDRDGRSGPLMDLVDALNPGTYRISFDTAKYSPGCF FPYVSIVFQVTESQKWEHFHVPLLLAPFSFSTYRGS | 6 |
| *Escherichia coli* | AQQNILSVHILNQQTGKPAADVTVTLEKKADNGWLQ LNTAKTDKDGRIKALWPEQTATTGDYRVVFKTGDYF KKQNLESFFPEIPVEFHINKVNEHYHVPLLLSQYGY STYRGS | 7 |
| *Bacillus subtilis* | MGKLTTHILDLTCGKPAANVKIGLKRLGESIMKEVY TNNDGRVDVPLLAGEELMSGEYVMEFHAGDYFASKN MNAADQPFLTIVTVRFQLADPDAHYHIPLLLSPFGY QVYRGS | 8 |
| *Herbaspirillum sp.* | MGKLSTHVLDITKGKPGVGVKLALYAVGPVGKTLLK QAVTNSDGRCDEPLLAGEALQVGKYELVFAAGDYFA AQGEQLPEPRFVDEVVIAFGIADASQNYHVPLVVSP WAYSTYRGS | 9 |
| *Deinococcus radiodurans* | MSGHPGLTTHVLDTARGKPAAGVRVQLCRVTGDTRT PVTEAVTNSDGRTDAPLIERGSLKQGTYELTFHVAD YFKGFVAAADPPFLDVVTLRFTVGDTSGHYHVPLVM TPWSYSTYRGS | 10 |
| *Klebsiella pneumoniae* | MSTLSTHILDISTGTPAEGVTVSLSREGETLANLVT NAQGRIATFSAAPLPAGRYCLTAETGAWFARAGRES VFTRAQIDFVIGEAAEDHFHLPFLIAPGGWSTYRGS | 11 |
| *Salmonella enterica* | AGNNILSVHILDQQTGKPAPGVEVVLEQKKDNGWTQ LNTGHTDQDGRIKALWPEKAAAPGDYRVIFKTGQYF ESKKLDTFFPEIPVEFHISKTNEHYHVPLLLSQYGY STYRGS | 12 |

(continued)

| Organism species | Amino acid sequence | SEQ ID NO |
|---|---|---|
| *Homo sapiens* (Transthyretin) | MASHRLLLLCLAGLVFVSEAGPTGTGESKCPLMVKV LDAVRGSPAINVAVHVFRKAADDTWEPFASGKTSES GELHGLTTEEEFVEGIYKVEIDTKSYWKALGISPFH EHAEVVFTANDSGPRRYTIAALLSPYSYSTTAVVTN PKE | 13 |

The highly conserved motif sequences in TTR and TRP are specifically the amino acid sequences represented by the following formula (I) (motif I) and formula (II) (motif II):

[K/H]-[I/V]-L-[D/N]-x-x-x-G-x-P-[A/G]-x-x-[L/I/V/M]-x-[I/V] ... (I)

[Y/W/F]-[T/H]-[I/V/T]-[A/P]-x-x-[L/I/V/M]-[S/T/A]-[P/Q]-[F/Y/W/G]-[G/S]-[F/Y]-[Q/S/T] ... (II)

wherein x represents any amino acid.

The HiUH for use in the uricase activator of the invention preferably comprises at least one amino acid sequence selected from the following amino acid sequences (i) and (ii):

(i) the amino acid sequence represented by motif I or motif II; and
(ii) an amino acid sequence which is different from any one amino acid sequence (i) with the substitution, deletion, or addition of one to three amino acids.

More preferably, the HiUH for use in the uricase activator of the invention comprises any combination of the following (i-1) and (i-2), (i-1) and (ii-2), (ii-1) and (ii-2), or (i-2) and (ii-1):

(i-1) the amino acid sequence represented by motif I;
(i-2) the amino acid sequence represented by motif II;
(ii-1) an amino acid sequence which is different from the amino acid sequence represented with motif I by the substitution, deletion, or addition of one to three amino acids; and (ii-2) an amino acid sequence which is different from the amino acid sequence represented with motif II by the substitution, deletion, or addition of one to three amino acids.

Figure 6 shows the molecular phylogenetic tree of HiUH derived from various organisms and human TTR which are shown in SEQ ID NOs: 1 to 13, wherein the molecular phylogenetic tree was prepared on the basis of the analysis of identity to the amino acid sequence (SEQ ID NO: 10) of *Deinococcus radiodurans* HiUH. The molecular phylogenetic tree was analyzed and prepared using Clustal Omega (https://www.ebi.ac.uk/Tools/msa/clustalo/) software on the basis of identity to the amino acid sequence represented by SEQ ID NO: 10. Tables 2 and 3 show sequences that correspond to motif I and motif II, respectively, in the amino acid sequences of HiUH derived from various organisms and human TTR which are shown in SEQ ID NOs: 1 to 4 and 6 to 13. In the tables, amino acids mismatched to the motifs are underlined. In the amino acid sequence (SEQ ID NO: 10) of *Deinococcus radiodurans* HiUH, motif I corresponds to positions 10 to 25, and motif II corresponds to positions 102 to 114. The amino acid sequence identity of HiUH between different species is less than 50% in all the cases, and the whole structure of HiUH is not highly conservative (Figure 6). However, it is evident that the motif sequences are highly conserved even between different species.

[Table 2]

| Organism species | Sequence |
|---|---|
| *Deinococcus radiodurans* | HLVDTARGKP AAGVRV |
| *Herbaspirillum seropedicae* | HVLDITKGKP GVGVKL |
| *Bacillus subtilis* | HVLDVSRGAP AAGVEV |
| *Mus musculus* | HVLNTGMGVP GAHMAL |
| *Brachiostoma japonicum* | HILDTSLGRP AADVPI |

(continued)

| Organism species | Sequence |
|---|---|
| *Danio rerio* | HVLNIAQGVP GANMTI |
| *Oncorhynchus kisutch* | HVLDTASGLP AQGLCL |
| *Arabidopsis thaliana* | HILDLTCGKP AANVKI |
| *Escherichia coli* | HILNQQTGKP AADVTV |
| *Klebsiella pneumoniae* | HILDISTGTP AEGVTV |
| *Salmonella enterica* | HILDQQTGKP APGVEV |
| *Homo sapiens* | KVLDAVRGSP AINVAV |

[Table 3]

| Organism species | Sequence |
|---|---|
| *Deinococcus radiodurans* | YHVPLVMTPW SYS |
| *Herbaspirillum seropedicae* | YHVPLWSPW AYS |
| *Bacillus subtilis* | YHIPLLLSPF GYQ |
| *Mus musculus* | FHVPLLLSPW SYT |
| *Brachiostoma japonicum* | YHVPLLLNPF GYS |
| *Danio rerio* | YHVPLLLSPF SYS |
| *Oncorhynchus kisutch* | FHVPLLCSRF SYT |
| *Arabidopsis thaliana* | FHVPLLLAPF SFS |
| *Escherichia coli* | YHVPLLLSQY GYS |
| *Klebsiella pneumoniae* | FHLPFLIAPG GWS |
| *Salmonella enterica* | YHVPLLLSQY GYS |
| *Homo sapiens* | YTIAALLSPY SYS |

In the uricase activator of the invention, any protein having activity of hydrolyzing HiU can be used as HiUH. Particularly, any protein having the motif sequence(s) described above can be used. For example, a protein having the amino acid sequence having 50% or higher, 60% or higher, 70% or higher, 80% or higher, 85% or higher, 90% or higher, 91% or higher, 92% or higher, 93% or higher, 94% or higher, 95% or higher, 96% or higher, 97% or higher, 98% or higher, or 99% or higher identity to the amino acid sequence represented by any of SEQ ID NOs: 1 to 12, and having activity of hydrolyzing HiU to produce OHCU can be used. In the specification, "identity" means sequence identity calculated using BLAST (Basic Local Alignment Search Tool at the National Center for Biological Information) or the like (e.g., default parameters, i.e., initially set parameters).

The HiUH particularly preferably has thermostability (is heat-resistant). For example, it is preferred that the HiUH should not be inactivated even if heat-treated at 60°C for 30 minutes. Examples of the organism of origin capable of producing such HiUH include bacteria of the genus *Bacillus,* the genus *Herbaspirillum,* and the genus *Deinococcus.*

The HiUH is further preferably HiUH having any of the following features (a) to (c):

(a) having the amino acid sequence of SEQ ID NO: 10;
(b) having an amino acid sequence having 70% or higher identity to the amino acid sequence of SEQ ID NO: 10, and having catalytic activity on the hydrolysis of HiU into OHCU; and
(c) having an amino acid sequence which is different from the amino acid sequence of SEQ ID NO: 10 with the deletion, substitution, or addition of one or several amino acids, and having catalytic activity on the hydrolysis of HiU into OHCU.

In the specification, the amino acid sequence of SEQ ID NO: 10 refers to a sequence identical to the amino

acid sequence of HiUH derived from *Deinococcus radiodurans* (hereinafter, also referred to as "DR"). In one embodiment of the uricase activator of the invention, the HiUH preferably has an amino acid sequence identical to that of HiUH derived from DR. Particularly, the HiUH derived from DR is HiUH confirmed to have a high uricase-activating effect as well as to have an effect of stabilizing uricase activity for a long period, and is suitably used in the uricase activator of the invention.

In another embodiment of the uricase activator of the invention, the HiUH preferably has 80% or higher, 85% or higher, 90% or higher, 91% or higher, 92% or higher, 93% or higher, 94% or higher, 95% or higher, 96% or higher, 97% or higher, 98% or higher, or 99% or higher identity to the amino acid sequence of SEQ ID NO: 10.

In a further alternative embodiment of the activator of the invention, the HiUH has an amino acid sequence having the deletion, substitution, or addition of one or several amino acids. In the specification, the term "several" of "several amino acids" refers to an integer of 2 to 10, preferably an integer of 2 to 6, more preferably an integer of 2 to 4, and further preferably an integer of 2 or 3.

In the uricase activator of the invention, recombinant HiUH produced by functionally integrating HiUH gene, for example, DNA having a nucleotide sequence encoding the amino acid sequence of a polypeptide having any of the features (a) to (c), in a host can be used as HiUH.

The type of the host to which the gene is transferred is not limited, and a single-celled eukaryote such as a bacterium, a fungus, or various yeasts, or animal or plant live cells can be arbitrarily selected. In the invention, a microbe is preferred, and *E. coli* is particularly preferred. The host *E. coli* is selected as an appropriate one from among *E. coli* K-12 strains that are usually used in gene engineering. Typical examples thereof include JM105 and JM109. DHS or, for example, BL21 or BL21 (DE3) for use in an inducible expression system may be used.

The HiUH gene is transferred through an expression vector which enhances the expression of the gene. The expression vector is a fusion product of the gene to be transferred with any of various DNA fragments or RNA fragments that enhance the expression thereof. Preferably, the expression vector may comprise a transcriptional promoter for constitutively or inducibly expressing the gene, a transcriptional terminator, and a selective marker. If desired, a cis element such as an enhancer, an operator, and a gene that controls a promoter may be comprised therein.

The vector is not limited, and a plasmid, such as pUC18, pUC19, pUC118, pUC119, pSC101, pBR322, pHSG298, pVC18, pVC19, pTrc99A, pMal-c2, pGEX2T, pTV118N, pTV119N, pTRP, or pET, which is often used for *E. coli* as a host can be preferably used. In addition, Yep13, Yep24, YCp50, pRS414, pRS415, pRS404, pAUR101, pKG1, or the like which is often used for *Saccharomyces cerevisiae* as a host can also be used, and a plasmid, such as pUB110 or pC194, which is often used for *Bacillus subtilis* as a host can also be used. Further, pBI122, pBI1101, or other various plasmids may be used without limitations.

The uricase activator of the invention comprises at least any HiUH described above and is used for highly activating uricase by contacting the HiUH with the uricase. The activator of the invention preferably increases the activity of uricase to 1.2 or more times, particularly, 1.5 or more times, and further 1.7 or more times. In the specification, 1 U of uricase refers to the amount of a catalyst that converts 1 $\mu$mol of uric acid into HiU per minute in measurement at 37°C.

The amount of the HiUH comprised in the uricase activator of the invention is preferably an amount that attains a weight concentration of 0.005 to 1.5 times, particularly, 0.01 to 1.0 times, and further 0.02 to 0.5 times the weight concentration of the uricase present together in terms of a final concentration.

The uricase activator of the invention may be used in uric acid measurement. In this case, the uricase activator may be comprised in advance as a portion of a uric acid measurement reagent comprising uricase, or may be used as a reagent different from a uric acid measurement reagent comprising uricase. For use in uric acid measurement, the activator of the invention may optionally comprise a phosphate buffer, a pH buffer such as Tris, MES, HEPES, or PIPES, a chelating agent such as EDTA, an antiseptic, or the like. Also, an enzyme stabilizer such as BSA, casein, or glycine may be comprised therein. The activator of the invention preferably has pH 5 to 9, particularly, pH 6 to 8, and further pH 6.5 to 7.5.

The uricase activator of the invention may be used as a constituent of a pharmaceutical composition for treating and/or preventing hyperuricemia or the like, comprising uricase (e.g., PEGylated uricase is suitably used). Alternatively, the activator may be used as a pharmaceutical composition that is used in combination with a pharmaceutical composition comprising uricase, aside from the pharmaceutical composition comprising uricase. A suitable content of HiUH in a pharmaceutical composition comprising the HiUH as an active ingredient, regardless of the presence or absence of uricase, differs depending on various conditions such as the type of the HiUH used, the ability to highly activate the uricase present together or the uricase used in combination, stability, the dosage form of the pharmaceutical composition, the type of a carrier used, an administration method, and the state of a recipient. These factors can be appropriately selected on the basis of a technique known in the art.

The pharmaceutical composition can optionally further comprise a pharmaceutically acceptable carrier. "Pharmaceutically acceptable carrier" refers to an additive that is usually used in the field of pharmaceutical technology. Examples thereof include excipients, binders, disintegrants, fillers, emulsifiers, flow modulators, and lubricants.

Examples of the excipients include sugars such as monosaccharides, disaccharides, cyclodextrin and polysaccharides (more specifically including, but not limited to, glucose, sucrose, lactose, raffinose, mannitol, sorbitol, inositol,

dextrin, maltodextrin, starch and cellulose), metal salts (e.g., sodium chloride, sodium phosphate or calcium phosphate, calcium sulfate, magnesium sulfate, and calcium carbonate), citric acid, tartaric acid, glycine, low-, medium- or high-molecular-weight polyethylene glycol (PEG), Pluronic(R), kaolin, silicic acid, and combinations thereof.

Examples of the binders include starch pastes using starch of corn, wheat, rice, or potato, simple syrup, glucose solutions, gelatin, tragacanth, methylcellulose, hydroxypropylmethylcellulose, carboxymethylcellulose sodium, shellac, and/or polyvinylpyrrolidone.

Examples of the disintegrants include the starch described above, lactose, carboxymethyl starch, crosslinked polyvinylpyrrolidone, agar, laminaran powders, sodium bicarbonate, calcium carbonate, alginic acid or sodium arginine, polyoxyethylene sorbitan fatty acid ester, sodium lauryl sulfate, monoglyceride stearate, and salts thereof.

Examples of the fillers include the sugars described above and/or calcium phosphate (e.g., tricalcium phosphate and calcium hydrogen phosphate).

Examples of the emulsifiers include sorbitan fatty acid ester, glycerin fatty acid ester, sucrose fatty acid ester, and propylene glycol fatty acid ester.

Examples of the flow modulators and the lubricants include silicate, talc, stearate, and polyethylene glycol.

Such a carrier is mainly used for easily forming the dosage form and maintaining the dosage form and a pharmacological effect and can be appropriately used, if necessary. A corrigent, a solubilizer, a suspending agent, a diluent, a surfactant, a stabilizer, an absorption promoter, an expander, a humectant, a moisturizing agent, an adsorbent, a disintegration inhibitor, a coating agent, a colorant, a preservative, an antioxidant, a fragrance, a flavoring agent, a sweetener, a buffer, or the like can also be comprised therein, if necessary, in addition to the additives described above.

The pharmaceutical composition can also comprise an additional drug without losing the pharmacological effect of the inhibitor. For example, an injection may comprise a predetermined amount of an antibiotic, an anti-inflammatory agent, or the like.

The dosage form of the pharmaceutical composition is not particularly limited as long as the form inactivates neither the inhibitor serving as an active ingredient nor other additional active ingredients. The dosage form may be, for example, a liquid, a solid, or a semi-solid. Specific examples of the dosage form include oral dosage forms such as solutions, powders, granules, tablets, capsules, sublingual formulations, and troches, and parenteral dosage forms such as injections, suspensions, emulsions, eye drops, nasal drops, creams, ointments, plasters, poultices, and suppositories.

The pharmaceutical composition can be administered by any appropriate method that does not inactivate the comprised active ingredient. For example, oral or parenteral administration (e.g., injection, aerosol, application, ocular instillation, and nasal instillation) may be used.

The pharmaceutical composition preferably comprises the HiUH serving as an active ingredient in an amount that is effective for the treatment and/or prevention of hyperuricemia or the like and does not cause serious adverse reaction.

The recipient of the pharmaceutical composition is a mammal such as a primate including a human and a chimpanzee, a pet animal such as a dog or a cat, a livestock animal such as a bovine, a horse, sheep, or a goat, a rodent such as a mouse or a rat, or an animal raised in a zoo, and is preferably a human. The recipient is more preferably a human in need of the treatment and/or prevention of hyperuricemia, gout, or the like.

Uric acid measurement and the treatment and/or prevention of hyperuricemia are exemplarily described above as use of the uricase activator of the present invention. However, the use of the uricase activator of the present invention is not limited thereto, and the uricase activator of the present invention can be employed in any use in need of uricase.

<Uricase activation method>

The uricase activation method of the invention has a feature of comprising the step of allowing the uricase activator of the present invention to be present together with uricase.

The method of the invention comprises at least the step of allowing the activator comprising HiUH to be present together with uricase. In the case of using uricase in uric acid measurement in a sample, the uricase and the uricase activator comprising HiUH may be mixed in advance and then mixed with the sample, or may be separately mixed with the sample. Uricase and HiUH, when separately mixed with the sample, may be concurrently mixed with the sample or may be mixed with the sample in a staggered manner.

In the case of activating uricase comprised in a pharmaceutical composition, the uricase and HiUH may be mixed into the same pharmaceutical composition so as to be present together *in vitro* before being administered to a subject in need of treatment and/or prevention. Alternatively, a pharmaceutical composition comprising uricase and a composition (preferably, a pharmaceutical composition) comprising HiUH may be separately administered to a subject in need of treatment and/or prevention so that the uricase and the HiUH are present together in the body of the subject.

The conditions, etc. of the uricase activation method of the invention are as described in the section <Uricase activator> unless otherwise specified.

<Uric acid measurement reagent>

The uric acid measurement reagent of the invention has a feature of comprising uricase and hydroxyisourate hydrolase and is used for measuring a uric acid concentration in a sample collected from a living body. The reagent of the invention has high uricase activity and is capable of measuring a wide concentration range of uric acid, by having the feature described above.

The uric acid measurement reagent of the invention is used for measuring a uric acid concentration in a sample collected from a living body. In this context, "living body" is a mammal such as a primate including a human and a chimpanzee, a pet animal such as a dog or a cat, a livestock animal such as a bovine, a horse, sheep, or a goat, a rodent such as a mouse or a rat, or an animal raised in a zoo, and is preferably a human. In the reagent of the invention, "sample" is a sample collected from a living body to be measured and can be appropriately selected from body fluids such as blood (including plasma and serum), lymph, urine, saliva, sweat, tissue fluid, body cavity fluid, and cerebrospinal fluid, and tissues. Among these samples, a body fluid, particularly, plasma, serum, or urine can be suitably used.

"Uricase" for use in the uric acid measurement reagent of the invention is an enzyme for uric acid as a substrate and is an enzyme that catalyzes reaction of degrading uric acid into HiU and hydrogen peroxide. Any uricase known in the art can be used as uricase for use in uric acid measurement as long as the enzyme has the catalytic activity described above. Uricase derived from any organism such as a vertebrate including a mammal, bird, and fish (however, naturally occurring uricase is known to be absent in primates), an invertebrate, a plant, a fungus (e.g., a yeast), and a bacterium may be used. For example, recombinant uricase produced by functionally integrating uricase gene derived from a bacterium or a fungus in a host (e.g., *E. coli*) may be used. In this case, the bacterium of origin or the fungus of origin is not particularly limited. For example, uricase derived from the genus *Bacillus,* a yeast, or the genus *Arthrobacter* may be used. Particularly, uricase derived from the genus *Bacillus* is preferred because of being excellent in stability.

The uricase for use in the uric acid measurement reagent of the invention may be an enzyme alone or may be an enzyme conjugated with a polymer such as polyethylene glycol (PEG). An enzyme alone is more preferred.

The uricase comprised in the reagent of the invention is not particularly limited, and any uricase that is used in a usual uric acid measurement reagent can be used. Particularly, a heat-resistant enzyme that is not inactivated even by treatment at 60°C for 30 minutes can be used.

In the uric acid measurement reagent of the invention, the HiUH is a hydrolytic enzyme that catalyzes reaction of hydrolyzing HiU into OHCU. The HiUH for use in the uric acid measurement reagent of the invention is not particularly limited by its organism of origin or structure as long as the enzyme has the activity described above. HiUH derived from any organism such as a vertebrate including a mammal, bird, and fish, an invertebrate, a plant, a fungus (e.g., a yeast), and a bacterium may be used. For example, the amino acid sequence of HiUH from a mouse (SEQ ID NO: 1), *Branchiostoma japonicum* (SEQ ID NO: 2), *Danio rerio* (SEQ ID NO: 3), or *Oncorhynchus kisutch* (SEQ ID NO: 4) for animals, the amino acid sequence of HiUH from *Glycine max* (SEQ ID NO: 5) or *Arabidopsis thaliana* (SEQ ID NO: 6) for plants, and the amino acid sequence of HiUH from *Escherichia coli* (SEQ ID NO: 7), *Bacillus subtilis* (SEQ ID NO: 8), a bacterium of the genus *Herbaspirillum* (SEQ ID NO: 9), *Deinococcus radiodurans* (SEQ ID NO: 10), *Klebsiella pneumoniae* (SEQ ID NO: 11), or *Salmonella enterica* (SEQ ID NO: 12) for bacteria have been reported.

The HiUH for use in the uric acid measurement reagent of the invention preferably comprises at least one amino acid sequence selected from the following amino acid sequences (i) and (ii):

(i) the amino acid sequence represented by the following formula (I) (motif I) or formula (II) (motif II):

[K/H]-[I/V]-L-[D/N]-x-x-x-G-x-P-[A/G]-x-x-[L/I/V/M]-x-[I/V] ... (I)

[Y/W/F]-[T/H]-[I/V/T]-[A/P]-x-x-[L/I/V/M]-[S/T/A]-[P/Q]-[F/Y/W/G]-[G/S]-[F/Y]-[Q/S/T] ... (II)

wherein x represents any amino acid; and

(ii) an amino acid sequence which is different from any one amino acid sequence (i) with the substitution, deletion, or addition of one to three amino acids.

More preferably, the HiUH for use in the uric acid measurement reagent of the present invention comprises any combination of the following (i-1) and (i-2), (i-1) and (ii-2), (ii-1) and (ii-2), or (i-2) and (ii-1):

(i-1) the amino acid sequence represented by motif I;
(i-2) the amino acid sequence represented by motif II;
(ii-1) an amino acid sequence which is different from the amino acid sequence represented with motif I by the substitution, deletion, or addition of one to three amino acids; and (ii-2) an amino acid sequence which is different

from the amino acid sequence represented by motif II with the substitution, deletion, or addition of one to three amino acids.

The HiUH comprised in the uric acid measurement reagent of the invention preferably has thermostability (is heat-resistant). For example, it is preferred that the HiUH should not be inactivated even if heat-treated at 60°C for 30 minutes. Examples of the organism of origin capable of producing such HiUH include bacteria of the genus *Bacillus,* the genus *Herbaspirillum,* and the genus *Deinococcus.*

The HiUH comprised in the reagent of the invention preferably has high specific activity and preferably has specific activity of 100 U/mg or more, particularly, 150 U/mg or more, and further 300 U/mg or more.

The HiUH comprised in the uric acid measurement reagent of the invention is preferably HiUH having any of the following features (a) to (c):

(a) having the amino acid sequence of SEQ ID NO: 10;
(b) having an amino acid sequence having 70% or higher identity to the amino acid sequence of SEQ ID NO: 10, and having catalytic activity on the hydrolysis of HiU into OHCU; and
(c) having an amino acid sequence which is different from the amino acid sequence of SEQ ID NO: 10 with the deletion, substitution, or addition of one or several amino acids, and having catalytic activity on the hydrolysis of HiU into OHCU.

In the specification, the amino acid sequence of SEQ ID NO: 10 refers to a sequence identical to the amino acid sequence of HiUH derived from *Deinococcus radiodurans* (hereinafter, also referred to as "DR"). In one embodiment of the uric acid measurement reagent of the invention, the HiUH preferably has an amino acid sequence identical to that of HiUH derived from DR. Particularly, the HiUH derived from DR is HiUH confirmed to have a high uricase-activating effect as well as to have an effect of stabilizing uricase for a long period, and is suitably used in the reagent of the invention.

In another embodiment of the uric acid measurement reagent of the invention, the HiUH preferably has 80% or higher, 85% or higher, 90% or higher, 91% or higher, 92% or higher, 93% or higher, 94% or higher, 95% or higher, 96% or higher, 97% or higher, 98% or higher, or 99% or higher identity to the amino acid sequence of SEQ ID NO: 10.

In a further alternative embodiment of the uric acid measurement reagent of the invention, the HiUH has an amino acid sequence which is different from the sequence with the deletion, substitution, or addition of one or several amino acids. In the specification, the term "several" of "several amino acids" refers to an integer of 2 to 10, preferably an integer of 2 to 6, more preferably 2 to 4, and further an integer of preferably 2 or 3.

In the reagent of the invention, recombinant HiUH produced by functionally integrating HiUH gene, for example, DNA having a nucleotide sequence encoding the amino acid sequence of a polypeptide having any of the features (a) to (c), in a host can be used as HiUH.

The type of the host to which the gene is transferred is not limited, and a single-celled eukaryote such as a bacterium, a fungus, or various yeasts, or animal or plant live cells can be arbitrarily selected. In the invention, a microbe is preferred, and *E. coli* is particularly preferred. The host *E. coli* is selected as an appropriate one from among *E. coli* K-12 strains that are usually used in gene engineering. Typical examples thereof include JM105 and JM109. DHS or, for example, BL21 or BL21 (DE3) for use in an inducible expression system may be used.

The HiUH gene is transferred through an expression vector which enhances the expression of the gene. The expression vector is a fusion product of the gene to be transferred with any of various DNA fragments or RNA fragments that enhance the expression thereof. Preferably, the expression vector may comprise a transcriptional promoter for constitutively or inducibly expressing the gene, a transcriptional terminator, and a selective marker. If desired, a cis element such as an enhancer, an operator, and a gene that controls a promoter may be comprised therein.

The vector is not limited, and a plasmid, such as pUC18, pUC19, pUC118, pUC119, pSC101, pBR322, pHSG298, pVC18, pVC19, pTrc99A, pMal-c2, pGEX2T, pTV118N, pTV119N, pTRP, or pET, which is often used for *E. coli* as a host can be preferably used. In addition, Yep13, Yep24, YCp50, pRS414, pRS415, pRS404, pAUR101, pKG1, or the like which is often used for *S. cerevisiae* as a host can also be used, and a plasmid, such as pUB110 or pC194, which is often used for *Bacillus subtilis* as a host can also be used. Further, pBI122, pBI1101, or other various plasmids may be used without limitations.

Exemplary reaction of a conventional uric acid measurement reagent is shown in the following formula I.

[Formula 1]

$$\text{Uric acid} + O_2 + H_2O \xrightarrow{\text{Uricase}} \text{Allantoin} + H_2O_2 + CO_2$$

$$2H_2O_2 + \text{4-AA} + \text{HDAOS} \xrightarrow{\text{POD}} 4H_2O + \text{Quinone dye} \quad \cdots\text{(III)}$$

The uric acid measurement reagent that employs the reaction of the formula (III) is capable of measuring a uric acid concentration by oxidatively degrading uric acid in a sample using uricase, and measuring the amount of the resulting hydrogen peroxide ($H_2O_2$). The amount of the generated $H_2O_2$ is measured through the oxidation reaction of peroxidase indicators by peroxidase (POD) activated by the $H_2O_2$. 4-aminoantipyrine (4-AA) and a modified Trinder's reagent such as N-(2-hydroxy-3-sulfopropyl)-3,5-dimethoxyaniline (HDAOS) are suitably used as the peroxidase indicators. A quinone dye resulting from the condensation reaction between 4-AA and the modified Trinder's reagent by peroxidase can be subj ected to colorimetry to measure the amount of the generated $H_2O_2$. For example, N-ethyl-N-sulfopropyl-3-methoxyaniline (ADPS), N-ethyl-N-sulfopropylaniline (ALPS), N-ethyl-N-sulfopropyl-3-methylaniline (TOPS), N-ethyl-N-(2-hydroxy-3-sulfopropyl)-3-methoxyaniline (ADOS), N-ethyl-N-(2-hydroxy-3-sulfopropyl)-3,5-dimethoxyaniline (DAOS), N-ethyl-N-(2-hydroxy-3 -sulfopropyl)-3,5 -dimethylaniline (MAO S), N-ethyl-N-(2-hydroxy-3 -sulfopropyl)-3 - methoxyaniline (TOOS), N,N-bis(4-sulfobutyl)-3,5-dimethylaniline (MADB), or N,N-bis(4-sulfobutyl)-3-methylaniline (TODB) may be used instead of HDAOS as the modified Trinder's reagent. Hereinafter, an aspect using 4-AA and HDAOS as the peroxidase indicators will be exemplarily described. However, this does not intend to limit the peroxidase indicators according to the present invention to 4-AA and HDAOS.

Usually, the uric acid measurement reagent has two test solutions, a first test solution comprising POD and HDAOS and a second test solution comprising uricase and 4-AA. A sample is mixed and incubated with the first test solution and then mixed and reacted with the second test solution to measure a uric acid concentration in the sample. The uric acid measurement reagent of the invention is not particularly limited as long as the reagent comprises uricase and particular HiUH and is capable of measuring uric acid. The reagent preferably comprises POD, HDAOS, and 4-AA. Further, ascorbic acid oxidase may be allowed to be present together therewith in order to suppress the influence of ascorbic acid in a specimen on a measured value. The uricase and the HiUH may be comprised in the same test solution or may be separately comprised in different test solutions. Particularly, an aspect having a first test solution comprising POD and HDAOS and a second test solution comprising uricase, HiUH, and 4-AA is preferred, though the aspect is not limited thereto.

The uric acid measurement reagent of the invention may optionally further comprise a phosphate buffer, a pH buffer such as Tris, MES, HEPES, or PIPES, a chelating agent such as EDTA, an antiseptic, or the like. Also, an enzyme stabilizer such as BSA, casein, or glycine may be comprised therein. The uric acid measurement reagent of the invention preferably has pH 5 to 9, particularly, pH 6 to 8, and further pH 6.5 to 7.5.

The uric acid measurement reagent of the invention preferably has a standard solution or a calibrator comprising a known concentration of uric acid, aside from the first test solution and the second test solution described above. The standard solution or the calibrator is concurrently measured with a sample to prepare a calibration curve, and the measured value of the sample can be applied to the calibration curve to calculate a uric acid concentration in the sample.

The uric acid measurement reagent of the invention, by comprising HiUH, preferably increases the activity of uricase to 1.2 or more times, particularly, 1.5 or more times, and further 1.7 or more times as compared with a uricase in a uric acid measurement reagent comprising no HiUH under the same conditions.

The uric acid measurement reagent of the invention preferably has high long-term stability. More specifically, it is also preferred that difference in uric acid measurement value calculated using the same calibration curve should be within 10% or within 5% in a reagent that has undergone an acceleration test at 37°C for 1 week or longer, particularly, 2 weeks or longer, and further 4 weeks or longer, as compared with a reagent without the acceleration test.

The amount of the HiUH comprised in the t reagent of present invention is preferably an amount that attains a weight concentration of 0.005 to 1.0 times, particularly, 0.01 to 0.5 times, and further 0.02 to 0.3 times the weight concentration of the uricase present together in terms of a final concentration. The active concentration of the HiUH comprised in the reagent of the invention is preferably 0.01 to 4.5 U/mL, particularly, 0.05 to 3.0 U/mL, in terms of a final concentration. The active concentration of the uricase comprised in the reagent of the invention is preferably 0.05 to 1.5 U/mL, particularly, 0.2 to 1.0 U/mL, in terms of a final concentration.

The active concentration of the POD comprised in a preferred form of the uric acid measurement reagent of the invention is preferably 1 to 5 U/mL, particularly, 2 to 4 U/mL, in terms of a final concentration. The concentration of the 4-AA is preferably 0.1 to 1.5 mM, particularly, 0.3 to 1.0 mM, in terms of a final concentration. The concentration of

the modified Trinder's reagent is preferably 0.1 to 1.5 mM, particularly, 0.3 to 1.0 mM, in terms of a final concentration.

Figure 1 shows a degradation reaction formula of uric acid by the uric acid measurement reagent of the invention. Uric acid comprised in a sample is reacted in the presence of uricase to produce HiU and hydrogen peroxide. For a conventional uric acid measurement reagent, the resulting HiU is nonenzymatically degraded into allantoin. In contrast, the uric acid measurement reagent of the invention further comprises HiUH, which also hydrolyzes HiU into OHCU through enzymatic reaction and subsequently degrades the OHCU into allantoin. Since the hydrolysis of HiU proceeds through nonenzymatic reaction as well as enzymatic reaction, the conversion of uric acid into HiU by uricase at the previous stage is also enhanced. The reagent of the invention is considered capable of measuring a wider concentration range of uric acid through the principles described above, as compared with a conventional uric acid measurement reagent.

An aspect using the uric acid measurement reagent of the invention will be exemplarily described. However, the scope in which the reagent of the invention can be used is not limited. When the reagent of the t invention has a first test solution comprising POD and HDAOS and a second test solution comprising uricase, HiUH, and 4-AA, an adopted method can involve mixing a sample with the first test solution, incubating the mixture at an optimum temperature (e.g., 37°C), and then mixing the mixture with the second reaction test solution for the reaction of the uricase and the HiUH. After the second test solution was mixed, the absorbance at 600 nm of the reaction system can be measured over time to calculate the amount of change (rate) in absorbance. At the same time, for example, absorbance at 800 nm can be measured to subsract a blank value. The operation described above may be carried out by a hand method using a microwell plate and can also be carried out using an automatic analyzer for clinical testing (e.g., Hitachi model 7180, manufactured by Hitachi, Ltd.).

The step of mixing and incubating a sample with the first test solution and the step of mixing and reacting the mixture with the second test solution are each preferably carried out under a temperature condition of 5.0 to 55°C, particularly, 20 to 45°C, and further 30 to 40°C. The required time of each step differs depending on the temperature condition, the concentration of each component for use in the reaction, etc. and is preferably 1 to 30 minutes, particularly, 2 to 10 minutes.

The uric acid measurement reagent of the invention configured as described above has high uricase activity and is thereby capable of measuring a uric acid concentration in a wider concentration range. The reagent of the invention has stability equivalent to or higher than that of a conventional product. The uric acid measurement reagent of the present invention is recommended to be refrigerated at approximately 4°C when not used. The uric acid measurement reagent is recommended to be used after being brought back to room temperature in advance upon use.

A detailed structure, characteristics, and preparation method, etc. of the HiUH for use in the reagent of the invention are as described in the section <Uricase activator> unless otherwise specified.

<Uric acid measurement method>

The uric acid measurement method of the invention has a feature of comprising the step of allowing the uric acid measurement reagent of the invention to be present together with a sample collected from a living body.

The uric acid measurement method of the invention requires allowing at least uricase and HiUH to be present together with a sample. The uricase and the HiUH may be concurrently mixed with the sample or may be sequentially mixed with the sample in a staggered manner.

The method of the invention may comprise, but not particularly limited to, the following steps.

First step: mixing and incubating a sample with a first test solution comprising POD and HDAOS.

Second step: mixing and reacting the mixed solution after the first step with a second test solution comprising uricase, HiUH, and 4-AA, and measuring the absorbance at 600 nm/800 nm of the mixed solution over time.

The uric acid measurement method of the invention may comprise the steps of: measuring signals derived from uric acid under the same conditions as to standard solutions comprising known concentrations of uric acid; and preparing a calibration curve.

The conditions, etc. of the method of the invention are as described in the section <Uric acid measurement reagent> unless otherwise specified.

Examples

Hereinafter, Examples of the invention will be described in order to describe the invention. However, these Examples do not intend to limit the scope of the invention to the scope of Examples.

<Test Example 1> Preparation of recombinant HiUH derived from *Deinococcus radiodurans. Bacillus subtilis,* and *Herbaspirillum seropedicae*

(1) Construction of each recombinant HiUH expression system

A nucleotide sequence encoding each of the amino acid sequence (SEQ ID NO: 10) of *Deinococcus radiodurans* (DR) HiUH, the amino acid sequence (SEQ ID NO: 8) of *Bacillus subtilis* (BS) HiUH, and the amino acid sequence (SEQ ID NO: 9) of *Herbaspirillum seropedicae* (HS) HiUH was optimized for *E. coli* codons to design HiUH gene derived from each bacterium. Each gene was designed so as to add a NdeI restriction site to the 5' end and a BamHI restriction site to the 3' end, and chemically synthesized. The obtained HiUH gene was treated with restriction enzymes NdeI and BamHI (manufactured by Takara Bio Inc.), and after agarose gel electrophoresis, recovered with GFX(TM) PCR DNA and Gel Band Purification Kit (manufactured by GE Healthcare Japan Corp). The recovered DNA fragment was inserted to an expression vector pET15b treated in advance with restriction enzymes NdeI and BamHI (manufactured by Takara Bio Inc.) using DNA Ligation Kit <Mighty Mix> (manufactured by Takara Bio Inc.). Subsequently, an *E. coli* JM109 strain was transformed therewith to obtain recombinant *E. coli* JM109(pET15b-DRHiUH), *E. coli* JM109(pET15b-BSHiUH), and *E. coli* JM109(pET15b-HSHiUH). Each recombinant thus obtained was shake-cultured at 37°C for 18 hours in LB medium comprising 50 μg/mL. Bacterial cells were recovered, and HiUH expression vectors pET15b-DRHiUH, pET15b-BSHiUH, and pET15b-HSHiUH were recovered using PureLink Quick Plasmid DNA Miniprep Kits (manufactured by Thermo Fisher Scientific Inc.). An *E. coli* BL21 (DE3) strain was transformed with each of these HiUH expression vectors to obtain recombinant *E. coli* BL21 (DE3)(pET15b-DRHiUH), *E. coli BL21* (DE3)(pET15b-BSHiUH), and A. *coli* BL21 (DE3)(pET15b-HSHiUH).

(2) Expression and purification of each recombinant HiUH

Each HiUH-expressing bacterial strain was inoculated to 100 mL of LB medium comprising 50 μg/mL ampicillin and then shake-cultured at 37°C until $OD_{600}$ reached 0.6 to 1.0. IPTG was added thereto at a final concentration of 0.1 mM, followed by shake culture at 30°C for 22 hours. Bacterial cells were recovered from the obtained culture solution by centrifugation (8,000 × g, 20 min).

The obtained wet bacterial cells of each strain were suspended in a 10 mM sodium borate buffer (pH 8.0) in an amount of 4 times the weight, sonicated, and then centrifuged (8,000 × g, 20 min), and a supernatant was recovered to obtain a crude extract. The obtained crude extract was applied to Ni-Sepharose 6FF (manufactured by GE Healthcare Japan Corp.) equilibrated with a 10 mM sodium borate buffer (pH 8.5) comprising 20 mM imidazole and 500 mM NaCl, and washed with the same buffer, followed by the elution of recombinant HiUH with a 10 mM sodium borate buffer (pH 8.5) comprising 500 mM imidazole and 500 mM NaCl. The obtained eluted fraction was buffer-replaced with a 10 mM sodium borate buffer (pH 8.5) comprising 1 mM DTT and 0.02% $NaN_3$ using PD-10 column (manufactured by GE Healthcare Japan Corp.), and the resultant was used as a purified product.

(3) Activity measurement of recombinant HiUH

The activity of the purified product of HiUH was measured by the following method: 1 mL of 100 mM $K\text{-}PO_4$ (pH 7.0) comprising 0.25 mM uric acid was placed in a cuvette and kept warm at 25°C for 5 minutes. Then, 4 μL of 7.2 U/mL rUricase (Y) (manufactured by Oriental Yeast Co., Ltd.) was added thereto, and the mixture was stirred by inverting, followed by absorbance measurement at 312 nm at 25°C. After 300 seconds from the start of measurement, 10 μL of the HiUH sample was added thereto, and the mixture was stirred by inverting, followed by measuring the change in absorbance measurement at 312 nm at 25°C. A blind test was conducted using a 100 mM $KH_2PO_4$ buffer (pH 7.5) comprising 0.5% BSA instead of the sample. The activity of HiUH was determined from a slope in the range of 420 to 480 seconds from the start of measurement according to the following expression.

[Expression 1]

$$\text{HiUH activity}=\frac{\Delta\text{Abs(420sec.-480sec.)-}\Delta\text{Abs Blind test(420sec.-480sec.)}\times\text{V}\times\text{D}}{\text{v}\times\text{A}}$$

wherein V: the final amount of the solution per quartz cuvette, v: the amount of the HiUH sample solution added to the quartz cuvette, A: a molar extinction coefficient (molar extinction coefficient at 312 nm (pH 7.0) of HiU: 8.5), and D: the dilution ratio of the enzyme sample solution)

1 U of HiUH was defined as the amount of a catalyst that hydrolyzes 1 μmol of HiU per minute in measurement at 25°C.

The measured activity value of each HiUH is shown in Table 4.

[Table 4]

| | DR | BS | HS |
|---|---|---|---|
| $A_{280}$ | 0.63 | 0.63 | 0.66 |
| U/mL | 193 | 74 | 122 |
| $U/A_{280}$ | 305 | 116 | 187 |

(4) Thermostability test of each recombinant HiUH

Each purified recombinant HiUH was heat-treated at 60°C for 30 minutes. The activity of the recombinant HiUH was measured before and after the heat treatment in the same manner as in (3), and the activity was compared between before and after the treatment. The ratio of the HiUH activity after the treatment to the HiUH activity before the treatment as to each recombinant HiUH is shown in Table 5. HiUH derived from DR exhibited the highest residual activity after the heat treatment.

[Table 5]

| | DR | BS | HS |
|---|---|---|---|
| Residual activity | 116.5% | 71.3% | 88.9% |

<Test Example 2> Preparation of recombinant uricase derived from the genus *Bacillus*

(1) Construction of recombinant uricase expression system

A gene optimized for *E. coli* codons, which encoded the amino acid sequence of uricase derived from the genus *Bacillus* (NCBI Accession No. BAA08723, SEQ ID NO: 14, Table 6) was designed so as to add a NdeI restriction site to the 5' end and a BamHI restriction site to the 3' end, and chemically synthesized. The obtained HiUH gene was treated with restriction enzymes NdeI and SalI (manufactured by Takara Bio Inc.), and after agarose gel electrophoresis, recovered with GFX(TM) PCR DNA and Gel Band Purification Kit (manufactured by GE Healthcare Japan Corp). The recovered DNA fragment was inserted to an expression vector pTRP2C treated in advance with restriction enzymes NdeI and SalI (manufactured by Takara Bio Inc.) using DNA Ligation Kit <Mighty Mix> (manufactured by Takara Bio Inc.). Subsequently, an *E. coli* JM109 strain was transformed therewith to obtain recombinant *E. coli* JM109(pTRP2C-UAO).

[Table 6]

| Organism species | Amino acid sequence | SEQ ID NO |
|---|---|---|
| *Bacillus subtilis* (Uricase) | MTKHKERVMYYGKGDVFAYRTYLKPLTGVRTIPESPFS<br>GRDHILFGVNVKISVGGTKLLTSFTKGDNSLVVATDSM<br>KNFIQKHLASYTGTTIEGFLEYVATSFLKKYSHIEKIS<br>LIGEEIPFETTFAVKNGNRAASELVFKKSRNEYATAYL | 14 |
| | NMVRNEDNTLNITEQQSGLAGLQLIKVSGNSFVGFIRD<br>EYTTLPEDSNRPLFVYLNIKWKYKNTEDSFGTNPENYV<br>AAEQIRDIATSVFHETETLSIQHLIYLIGRRILERFPQ<br>LQEVYFESQNHTWDKIVEEIPESEGKVYTEPRPPYGFQ<br>CFTVTQEDLPHENILMFSDEPDHKGALK | |

(2) Expression and purification of recombinant uricase

The uricase-expressing bacterial strain was inoculated to 100 mL of LB medium comprising 50 μg/mL ampicillin and then shake-cultured at 37°C for 15 to 18 hours until $OD_{600}$ reached 3.0. Subsequently, the culture solution was

added to 1.5 L of LB medium (comprising 0.05 g/L ampicillin, pH 7.4) and feeding-cultured at 37°C for 8 hours by the addition of 300 mL of a feeding medium (comprising 150 g/L yeast extract, 2 g/L $MgSO_4$, and 100 g/L glucose).

Bacterial cells were collected from the culture solution, and 250 g of the obtained wet bacterial cells was suspended in a 100 mM borate buffer (pH 9.0). The bacterial cells were disrupted using small Dynomill and heat-treated at 55°C for 30 minutes. The uricase sample thus heat-treated was purified by ion-exchange chromatography and hydrophobic chromatography to obtain purified uricase. The specific activity of the purified uricase was 4.1 U/mg of protein.

<Test Example 3> Comparison of uricase-activating effect of each recombinant HiUH

A U solution comprising 1 U/mL recombinant uricase derived from the genus *Bacillus* (hereinafter, also referred to as "uricase(B)") prepared in Test Example 2 or commercially available rUricase (yeast-derived, manufactured by Oriental Yeast Co., Ltd., specific activity: 39.3 U/mg of protein) (hereinafter, also referred to as "uricase(Y)"), or a U + H solution comprising 1 U/mL each uricase and 5 U/mL each recombinant HiUH was mixed into a 0.2 M $KH_2PO_4$ buffer (pH 7.0) at the quantitative ratio shown in Table 7 to prepare each uricase reaction test solution.

[Table 7]

| | HiUH activity (U/mL) | | | | |
|---|---|---|---|---|---|
| | 0 | 0.5 | 1.0 | 2.5 | 5.0 |
| U solution | 500 μL | 450 μL | 400 μL | 250 μL | - |
| U+H solution | - | 50 μL | 100 μL | 250 μL | 500 μL |

A uric acid solution was prepared as a sample for measurement. Uric acid (manufactured by FUJIFILM Wako Pure Chemical Corp.) was diluted with a 0.2 M $KH_2PO_4$ buffer (pH 7.0) to prepare a 0.125 mM uric acid solution, which was used as a sample for measurement.

3 mL of each uricase reaction test solution was added to a cell of a spectrophotometer (UV1800, manufactured by Shimadzu Corp.) and kept warm for 5 minutes in a thermostat of 37°C. 20 μL of the sample for measurement was added to the cell kept warm, and the mixture was quickly mixed by inverting. Rate of change in absorbance, Δmab, at 293 nm was calculated 60 to 120 seconds after the start of measurement. When ΔmAbs at a HiUH concentration of zero was defined as 100%, a relative value (%) of the amount of change of ΔmAbs of each uricase reaction test solution was calculated. The uricase activity of each uricase reaction test solution is shown in Figure 2. Figure 2(A) shows the influence of HiUH on the activity of the uricase(B). Figure 2(B) shows the influence of HiUH on the activity of the uricase(Y). Activation by the addition of HiUH was found in both the uricases. Particularly, use of HiUH derived from DR was shown to activate uricase.

<Test Example 4> Verification of effect of addition of HiUH derived from DR on uric acid measurement reagent

The following measurement reagents 1 and 2 were provided using a commercially available uric acid measurement reagent "Serotec" UA-L (manufactured by Serotec Co,. Ltd.).

Measurement reagent 1: Uricase of R-2 in "Serotec" UA-L was replaced with the recombinant uricase prepared in Test Example 2. R-2 was filtered through an ultrafiltration filter (Amicon Ultra 15, 10k membrane, manufactured by Merck KGaA), and the recombinant uricase prepared in Test Example 2 was added to the recovered filtrate. The added recombinant uricase had the same amount of activity as that of the uricase originally used in the product.

Measurement reagent 2: The HiUH derived from DR prepared in Test Example 1 was further added to R-2 of measurement reagent 1. The added HiUH had twice the amount of activity of uricase.

Uric acid (manufactured by FUJIFILM Wako Pure Chemical Corp.) was dissolved in a 50 mM borate buffer (pH 8.5) to prepare a 200 mg/dL uric acid solution. Subsequently, the uric acid solution was serially diluted with the same buffer thereas to prepare 200, 180, 160, 140, 120, 100, 80, 60, 40, 20, 10, 5, and 0 mg/dL uric acid dilution series. Aside from the uric acid dilution series, a uric acid calibrator (10 mg/dL, manufactured by FUJIFILM Wako Pure Chemical Corp.) was provided.

The uric acid dilution series and the uric acid calibrator were measured using measurement reagents 1 and 2 and an automatic analyzer 7180 (manufactured by Hitachi High-Tech Corp.). The measurement conditions in the automatic analyzer 7180 were set in accordance with the conditions described in the package insert of "Serotec" UA-L (10th edition, issued in August, 2017) except that the amount of a specimen was changed from 3.2 μL to 4.8 μL.

The amount of change in absorbance at 600 nm/800 nm at a measurement point 16-34 was determined for each solution of the uric acid dilution series and the uric acid calibrator. A calibration curve was prepared from the

amounts of change in absorbance of a uric acid concentration of 0 mg/dL and the uric acid calibrator, and an actually measured concentration of each solution was calculated from the amount of change in absorbance of each of the uric acid dilution series using this calibration curve. Figure 3 shows a graph in which a theoretical concentration value of uric acid (abscissa) was plotted against a measured concentration value measured with the measurement reagent comprising HiUH or comprising no HiUH (ordinate) for each solution of the uric acid dilution series. Figure 4 shows a graph in which a calculated ratio (%) of a measured concentration value measured with the measurement reagent comprising HiUH or comprising no HiUH to a theoretical concentration value of uric acid was plotted against the theoretical value as to each solution of the uric acid dilution series. Provided that measured value/theoretical value (%) of the uric acid concentration was 100% ± 2.5%, this measurement was defined as being accurate and the dilutional linearity of each measurement reagent was evaluated. As a result, the dilutional linearity was limited to the uric acid concentration up to 150 mg/dL for measurement reagent 1 comprising no HiUH, whereas the dilutional linearity was found at the uric acid concentration up to 270 mg/dL for measurement reagent 2 comprising uricase present together with HiUH. The addition of HiUH together with uricase to the uric acid measurement reagent was able to widen the measurable range of the uric acid concentration.

<Test Example 5> Stability test of uric acid measurement reagent

The following R-2(1) to R-2(3) were prepared using a commercially available uric acid measurement reagent "Serotec" UA-L (manufactured by Serotec Co., Ltd.).

R-2(A) (control): Uricase of R-2 in "Serotec" UA-L was replaced with the recombinant uricase prepared in Test Example 2. R-2 was filtered through an ultrafiltration filter (Amicon Ultra 15, 10k membrane, manufactured by Merck KGaA), and the recombinant uricase prepared in Test Example 2 was added to the recovered filtrate. The added recombinant uricase had the same amount of activity as that of the uricase originally used in the product.

R-2(B): The HiUH derived from DR prepared in Test Example 1 was further added to R-2 of measurement reagent 1. The added HiUH had twice the amount of activity of uricase.

R-2(C): The HiUH derived from DR prepared in Test Example 1 was further added to R-2 of measurement reagent 1. The added HiUH had three times the amount of activity of uricase.

The concentrations of the uricase and the HiUH comprised in each R-2 are shown in Table 8.

[Table 8]

| | Specific activity (U/mg) | R-2(A) (Control) (U/mL) | R-2(B) (U/mL) | R-2(C) (U/mL) |
|---|---|---|---|---|
| Recombinant uricase | 4.1 | 0.8 U (3.2 mg) | 0.8 U (3.2 mg) | 0.8 U (3.2 mg) |
| Recombinant HiUH | 305 | - | 1.6 U (0.005 mg) | 2.4 U (0.008 mg) |
| HiUH/uricase weight ratio | | 0 | 0.016 | 0.025 |

Each R-2 was left standing for periods of 2 weeks or 4 weeks in a thermostat of 37°C (acceleration test). 200, 180, 160, 140, 120, 100, 80, 60, 40, 20, 10, 5, and 0 mg/dL uric acid dilution series were measured in the same manner as in Test Example 4 using each R-2 and R-1 of "Serotec" UA-L. Uric acid calibration was measured using each R-2 without the acceleration test to prepare a calibration curve under the conditions of each R-2.

The amount of change in absorbance at 600 nm/800 nm at a measurement point 16-34 was determined for each solution of the uric acid dilution series and the uric acid calibrator. A calibration curve was prepared from the amounts of change in absorbance of a uric acid concentration of 0 mg/dL and the uric acid calibrator, and a theoretical value of the amount of change in absorbance was calculated using this calibration curve. Figure 5 shows a graph in which a theoretical concentration value of uric acid (abscissa) was plotted against a measured value of change in absorbance measured using the reagent after implementation of each acceleration test (ordinate) as to each solution of the uric acid dilution series. Figure 5A shows a measured value of R-2(A), i.e., the control. Figure 5B shows a measured value obtained using R-2(B), i.e., the reagent comprising HiUH at twice the active concentration of uricase. Figure 5C shows a measured value obtained using R-2(C), i.e., the reagent comprising HiUH at three times the active concentration of uricase. The measured value of a high concentration of uric acid tended to be decreased in response to the length of the acceleration test for the control reagent, whereas high dilutional linearity of the uric acid measurement value was maintained even after the acceleration test for both the reagents comprising HiUH.

Industrial Applicability

The present invention is applicable mainly to the industrial fields of clinical examination, *in vitro* diagnostic

medicaments, and medicaments.

All publications, patents, and patent applications cited herein are incorporated herein by reference in their entirety.

**Claims**

1. A uricase activator comprising hydroxyisourate hydrolase.

2. The activator according to claim 1, wherein the hydroxyisourate hydrolase comprises at least one amino acid sequence selected from the following amino acid sequences (i) and (ii), and has catalytic activity on the hydrolysis of 5-hydroxyisourate into 2-oxo-4-hydroxy-4-carboxy-5 -ureidoimidazoline:

   (i) an amino acid sequence represented by the following formula (I) or (II):

   [K/H]-[I/V]-L-[D/N]-x-x-x-G-x-P-[A/G]-x-x-[L/I/V/M]-x-[I/V] ... (I)

   [Y/W/F]-[T/H]-[I/V/T]-[A/P]-x-x-[L/I/V/M]-[S/T/A]-[P/Q]-[F/Y/W/G]-[G/S]-[F/Y]-[Q/S/T] ... (II)

   wherein x represents any amino acid, and
   (ii) an amino acid sequence which is different from any one amino acid sequence (i) with the substitution, deletion, or addition of one to three amino acids.

3. The activator according to claim 1 or 2, wherein the uricase activator is used for activating uricase by allowing the uricase activator to be present together with the uricase, and a content of the hydroxyurate hydrolase is 0.005 to 1.5 times an amount of the uricase present together in terms of a weight ratio.

4. The activator according to any one of claims 1 to 3, wherein the uricase activator increases the activity of uricase to 1.2 or more times.

5. The activator according to any one of claims 1 to 4, wherein the hydroxyisourate hydrolase is a thermostable enzyme.

6. The activator according to any one of claims 1 to 5, wherein specific activity of the hydroxyisourate hydrolase is 100 U/mg or more.

7. The activator according to any one of claims 1 to 6, wherein the hydroxyisourate hydrolase is derived from the genus *Bacillus,* the genus *Herbaspirillum,* or the genus *Deinococcus.*

8. The activator according to any one of claims 1 to 6, wherein the hydroxyisourate hydrolase has any of the following features (a) to (c):

   (a) having the amino acid sequence of SEQ ID NO: 10;
   (b) having an amino acid sequence having 70% or higher identity to the amino acid sequence of SEQ ID NO: 10, and having catalytic activity on the hydrolysis of 5-hydroxyisourate into 2-oxo-4-hydroxy-4-carboxy-5-ureidoimidazoline; and
   (c) having an amino acid sequence which is different from the amino acid sequence of SEQ ID NO: 10 with the deletion, substitution, or addition of one or several amino acids, and having catalytic activity on the hydrolysis of 5-hydroxyisourate into 2-oxo-4-hydroxy-4-carboxy-5-ureidoimidazoline.

9. The activator according to any one of claims 1 to 8, wherein the hydroxyisourate hydrolase is derived from *Deinococcus radiodurans.*

10. A uricase activation method comprising the step of allowing uricase and a uricase activator according to any one of claims 1 to 9 to be present together.

11. The method according to claim 10, wherein the step is the step of allowing the uricase and the uricase activator to be present together at a weight ratio of from 1:0.005 to 1:1.5.

**12.** A uric acid measurement reagent for use in measuring a uric acid concentration in a sample collected from a living body, comprising uricase and hydroxyisourate hydrolase.

**13.** The reagent according to claim 12, wherein the hydroxyisourate hydrolase comprises at least one amino acid sequence selected from the following amino acid sequences (i) and (ii), and has catalytic activity on the hydrolysis of 5-hydroxyisourate into 2-oxo-4-hydroxy-4-carboxy-5 -ureidoimidazoline:

(i) an amino acid sequence represented by the following formula (I) or (II):

[K/H]-[I/V]-L-[D/N]-x-x-x-G-x-P-[A/G]-x-x-[L/I/V/M]-x-[I/V] ... (I)

[Y/W/F]-[T/H]-[I/V/T]-[A/P]-x-x-[L/I/V/M]-[S/T/A]-[P/Q]-[F/Y/W/G]-[G/S]-[F/Y]-[Q/S/T] ... (II)

wherein x represents any amino acid, and
(ii) an amino acid sequence which is different from any one amino acid sequence (i) with the substitution, deletion, or addition of one to three amino acids.

**14.** The reagent according to claim 12 or 13, wherein a weight concentration ratio of the uricase to the hydroxyurate hydrolase is from 1:0.005 to 1:1.5.

**15.** The reagent according to any one of claims 12 to 14, wherein the hydroxyisourate hydrolase is a thermostable enzyme.

**16.** The reagent according to any one of claims 12 to 15, wherein specific activity of the hydroxyisourate hydrolase is 100 U/mg or more.

**17.** The reagent according to any one of claims 12 to 16, wherein the hydroxyisourate hydrolase is derived from the genus *Bacillus,* the genus *Herbaspirillum,* or the genus *Deinococcus.*

**18.** The reagent according to any one of claims 12 to 17, wherein the hydroxyisourate hydrolase has any of the following features (a) to (c):

(a) having the amino acid sequence of SEQ ID NO: 10;
(b) having an amino acid sequence having 70% or higher identity to the amino acid sequence of SEQ ID NO: 10, and having catalytic activity on the hydrolysis of 5-hydroxyisourate into 2-oxo-4-hydroxy-4-carboxy-5-ureidoimidazoline; and
(c) having an amino acid sequence which is different from the amino acid sequence of SEQ ID NO: 10 with the deletion, substitution, or addition of one or several amino acids, and having catalytic activity on the hydrolysis of 5-hydroxyisourate into 2-oxo-4-hydroxy-4-carboxy-5-ureidoimidazoline.

**19.** The reagent according to any one of claims 12 to 18, wherein the hydroxyisourate hydrolase is derived from *Deinococcus radiodurans.*

**20.** A method comprising the step of mixing and reacting a uric acid measurement reagent according to any one of claims 12 to 19 with a sample collected from a living body.

Fig. 1

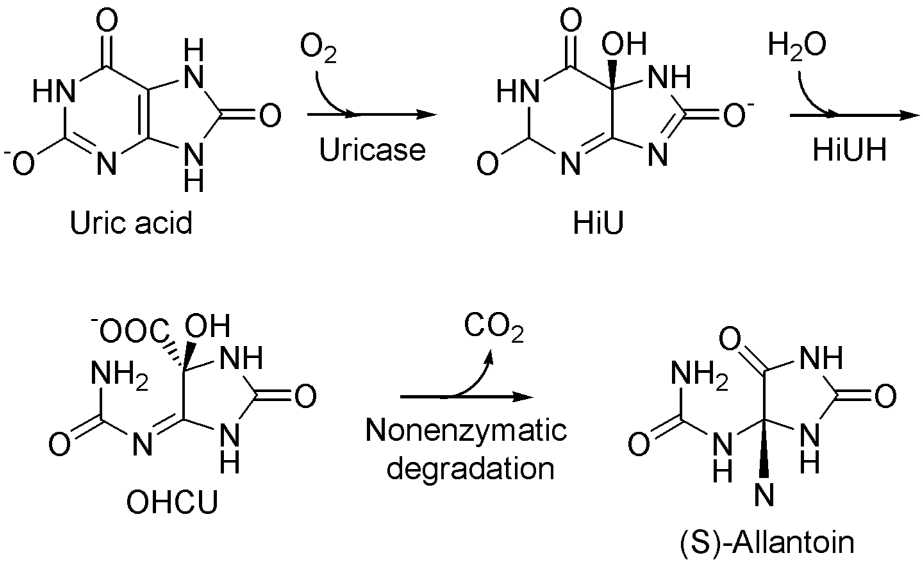


Uric acid

HiU

OHCU

(S)-Allantoin

Fig. 2

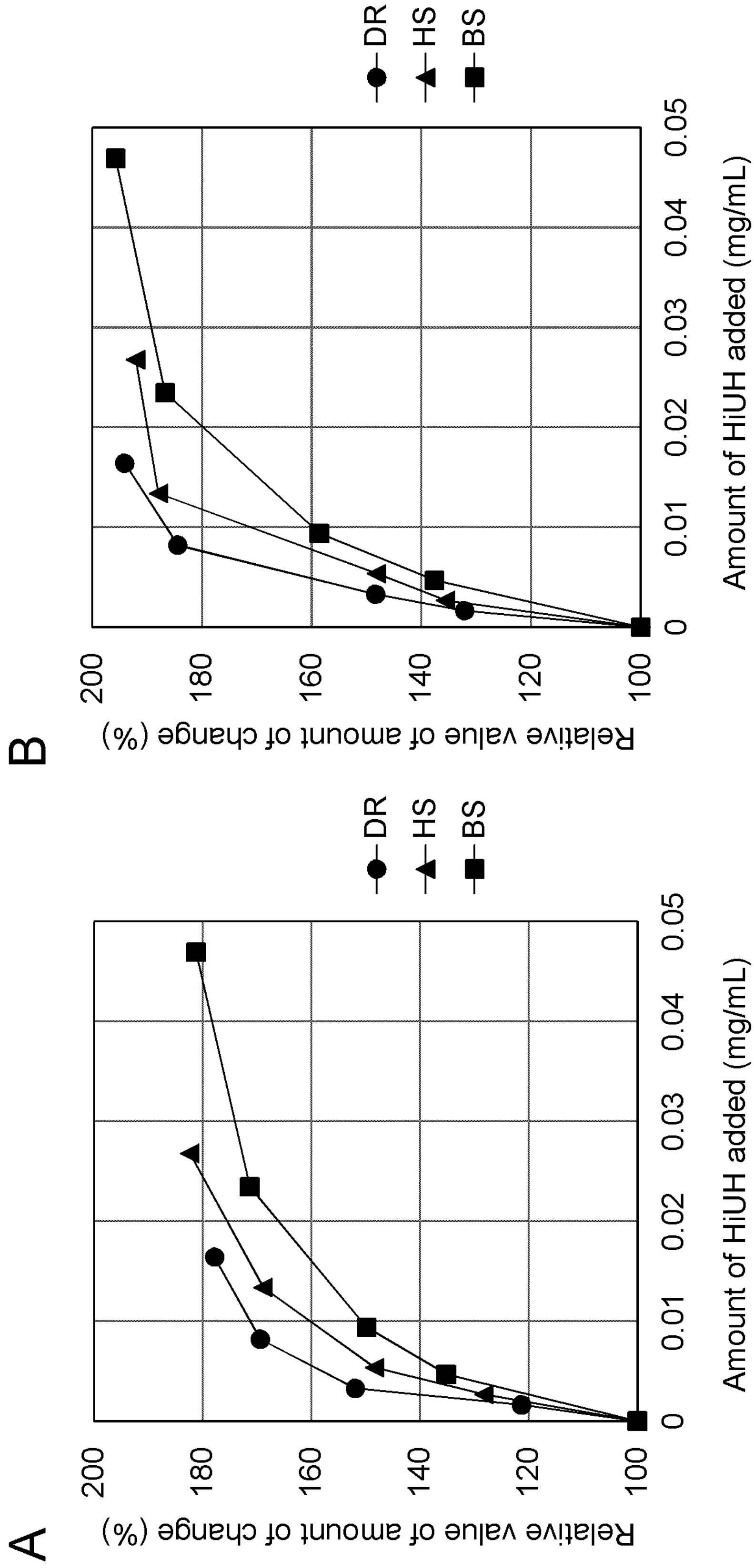
A
B
Relative value of amount of change (%)
200
180
160
140
120
100
0
0.01
0.02
0.03
0.04
0.05
Amount of HiUH added (mg/mL)
DR
HS
BS

Fig. 3

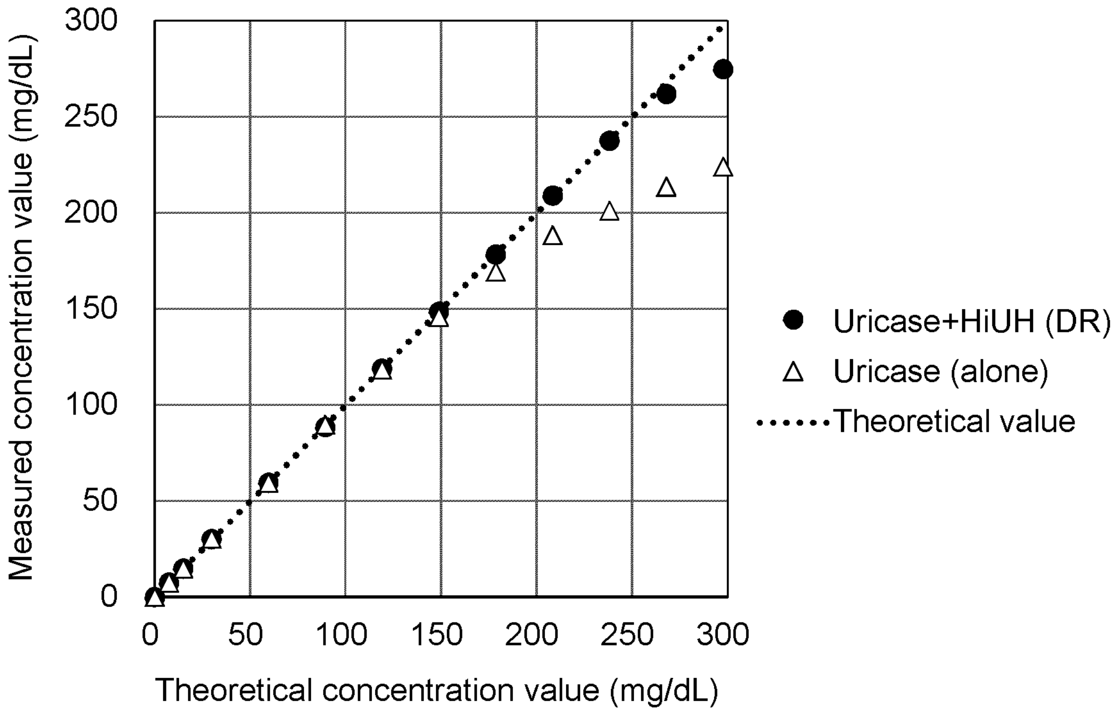
Measured concentration value (mg/dL)
300
250
200
150
100
50
0
0
50
100
150
200
250
300
Theoretical concentration value (mg/dL)
Uricase+HiUH (DR)
Uricase (alone)
Theoretical value

Fig. 4

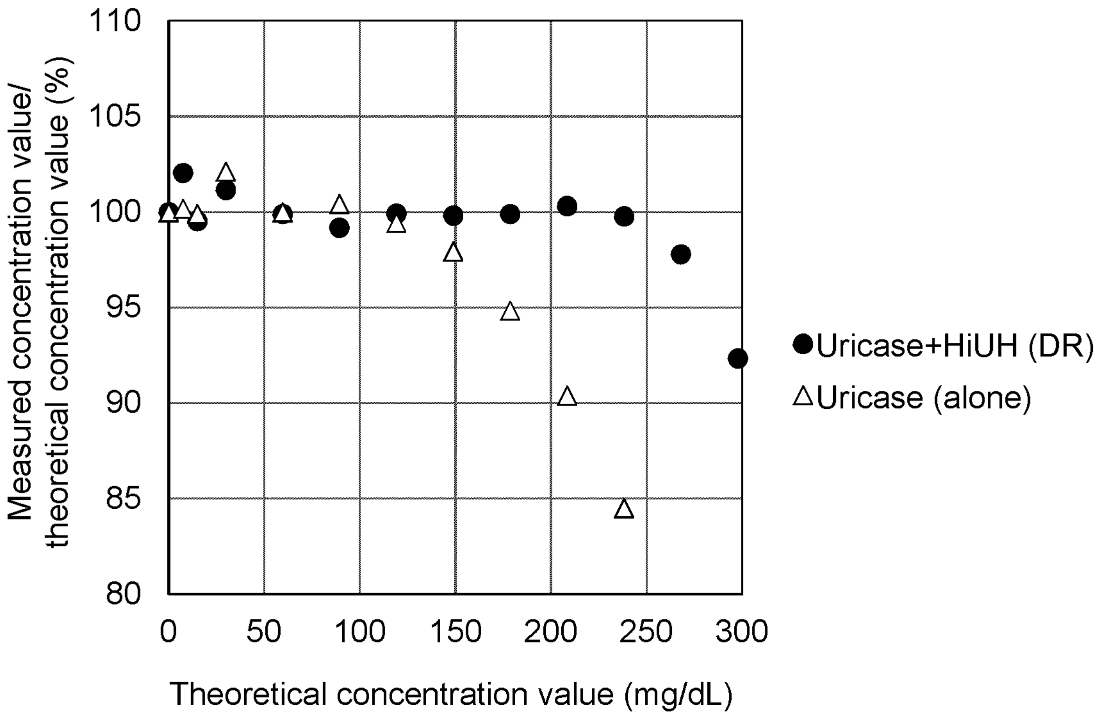
110
105
100
95
90
85
80
Measured concentration value/
theoretical concentration value (%)
0
50
100
150
200
250
300
Theoretical concentration value (mg/dL)
●Uricase+HiUH (DR)
△Uricase (alone)

Fig. 5

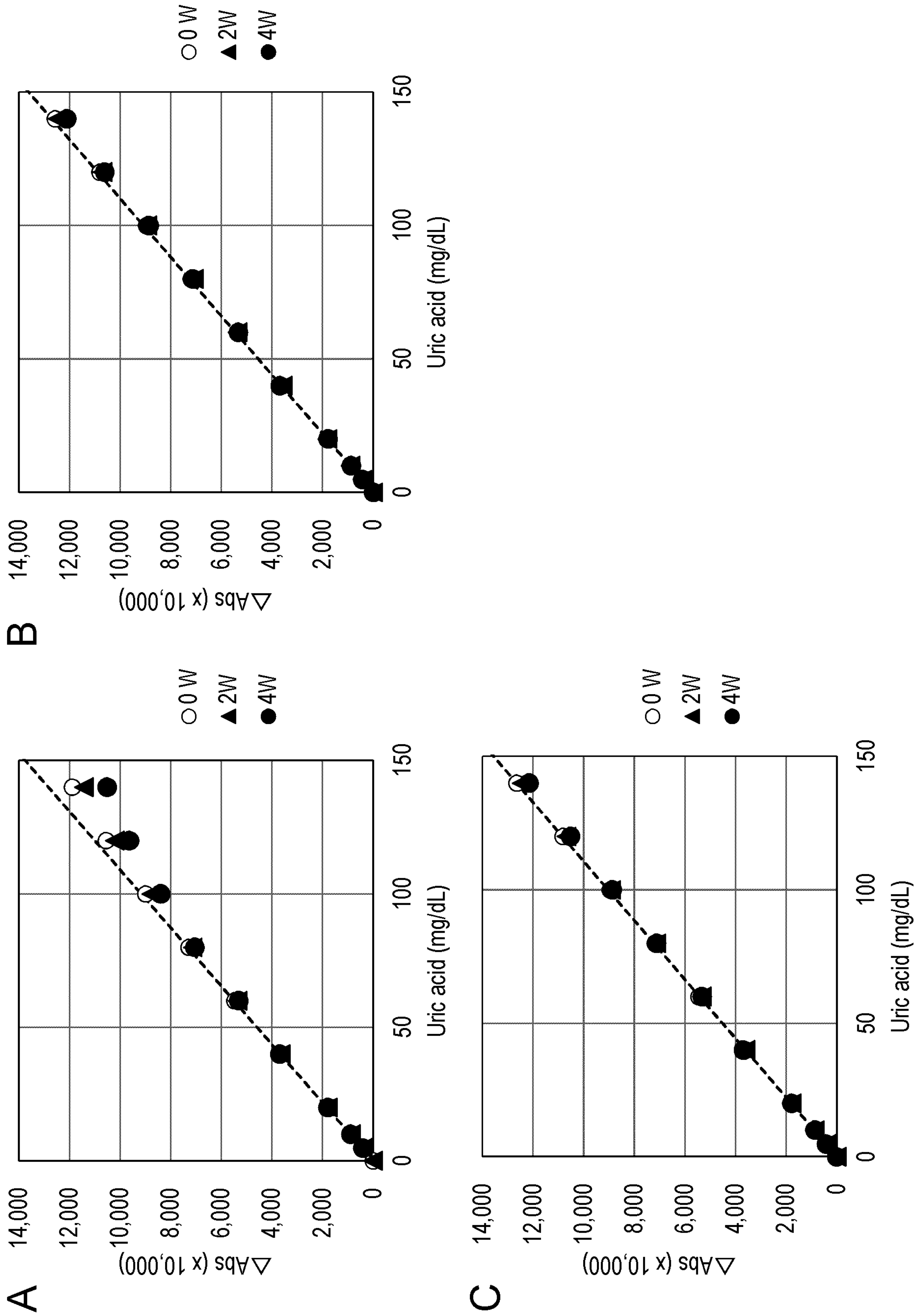
A
B
C
ΔAbs (x 10,000)
0
2,000
4,000
6,000
8,000
10,000
12,000
14,000
Uric acid (mg/dL)
0
50
100
150
○0 W
▲2W
●4W

Fig. 6

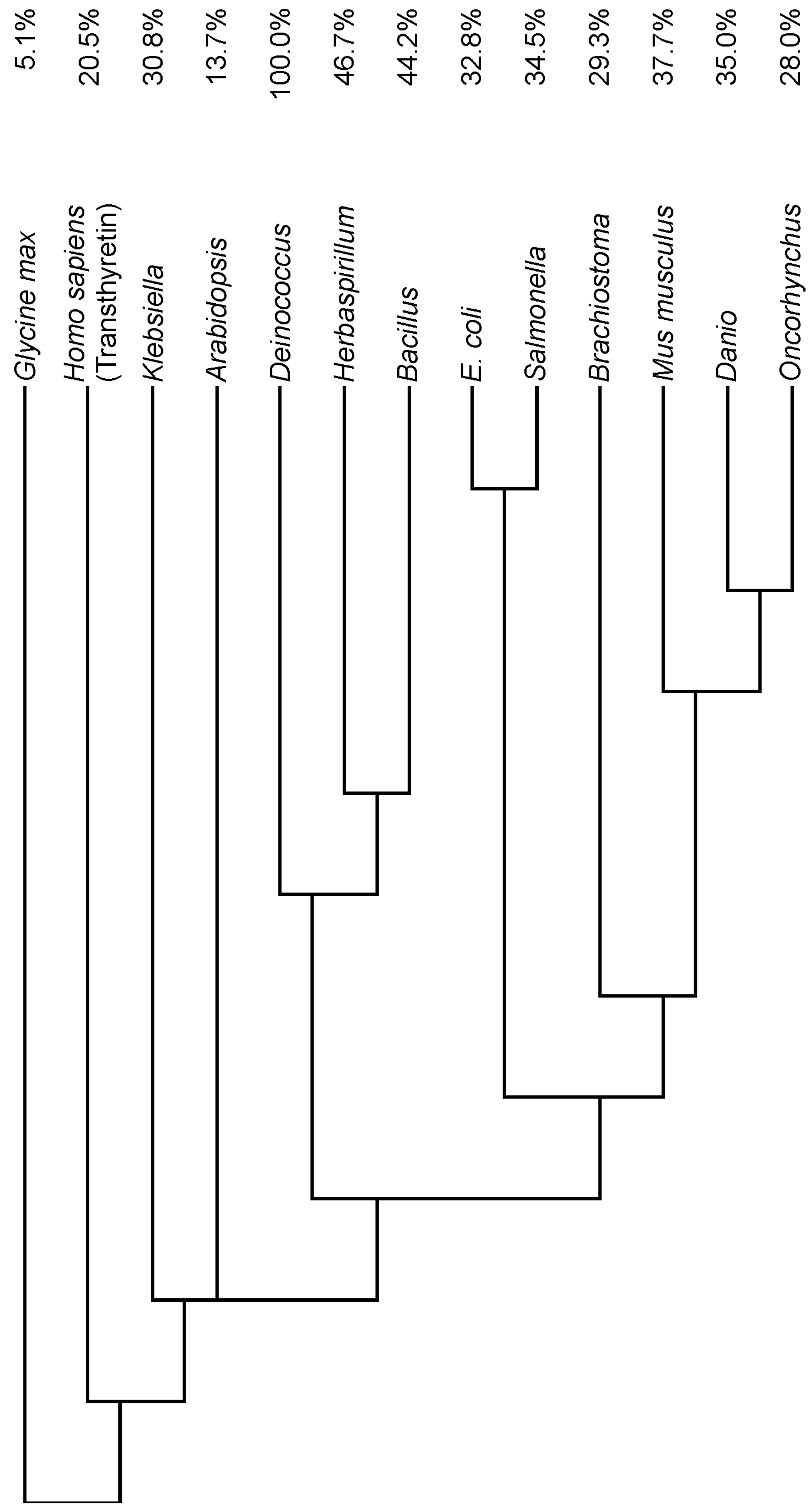
Glycine max 5.1%
Homo sapiens (Transthyretin) 20.5%
Klebsiella 30.8%
Arabidopsis 13.7%
Deinococcus 100.0%
Herbaspirillum 46.7%
Bacillus 44.2%
E. coli 32.8%
Salmonella 34.5%
Brachiostoma 29.3%
Mus musculus 37.7%
Danio 35.0%
Oncorhynchus 28.0%

**INTERNATIONAL SEARCH REPORT**

International application No.
PCT/JP2021/004869

A. CLASSIFICATION OF SUBJECT MATTER
Int.Cl. C12N15/55(2006.01)n, C12Q1/26(2006.01)i, C12Q1/34(2006.01)i, C12N9/06(2006.01)i, C12N9/14(2006.01)i, G01N33/50(2006.01)i
FI: C12N9/14ZNA, C12N9/06A, C12Q1/26, C12Q1/34, G01N33/50A, C12N15/55
According to International Patent Classification (IPC) or to both national classification and IPC

B. FIELDS SEARCHED
Minimum documentation searched (classification system followed by classification symbols)
Int.Cl. C12N1/00-15/90, C12Q1/00-3/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580(JDreamIII), CAplus/MEDLINE/EMBASE/BIOSIS/(STN), UniProt/GeneSeq, PubMed

C. DOCUMENTS CONSIDERED TO BE RELEVANT

| Category* | Citation of document, with indication, where appropriate, of the relevant passages | Relevant to claim No. |
|---|---|---|
| X<br>Y | LEE, Y. et al., Transthyretin-related proteins function to facilitate the hydrolysis of 5-hydroxyisourate, the end product of the uricase reaction, FEBS Lett., 2005, vol. 579, pp. 4769-4774, particularly, p. 4769, left column, abstract, p. 4769, right column, l. 3 to p. 4770, left column, l. 3, p. 4771, right column, l. 1 to l. 9 from the bottom, fig. 1, 3, 4 | 1, 3-4, 6-7, 10-11<br>1-20 |
| Y | WO 2007/052326 A2 (UNIVERSITA DEGLI STUDI DI PARMA) 10 May 2007 (2007-05-10), column 6, lines 1-8, column 14, line 14 to column 15, line 6, fig. 5 | 1-20 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 March 2021 | 13 April 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No. PCT/JP2021/004869

C (Continuation). DOCUMENTS CONSIDERED TO BE RELEVANT

| Category* | Citation of document, with indication, where appropriate, of the relevant passages | Relevant to claim No. |
|---|---|---|
| Y | フレックスカートリッジ尿酸 URCA, シーメンスヘルスケア・ダイアグノスティクス株式会社, 2017, particularly, left column, 「全般的な注意」-「測定原理」の項, non-official translation (Flex Cartridge Uric Acid URCA, SIEMENS HEALTHCARE DIAGNOSTICS INC., item of "General precautions" to "Measurement principle") | 1-20 |

Form PCT/ISA/210 (second sheet) (January 2015)

| INTERNATIONAL SEARCH REPORT<br>Information on patent family members | International application No.<br>PCT/JP2021/004869 |
| --- | --- |

WO 2007/052326 A2 10 May 2007 (Family: none)

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002522399 A
- WO 2006030866 A
- JP 6070798 A
- JP 6038766 A
- WO 2007052326 A

### Non-patent literature cited in the description


- **Y. LEE et al.** *FEBS Letters,* 2005, vol. 579, 4769-4774
- **K. YAMAUCHI ; K. KASAI.** *J. Mol. Evol.,* 2018, vol. 86, 457-469
- **G. ZANOTTI et al.** *J. Mol. Biol.,* 2006, vol. 363, 1-9
- **A. RAYCHAUDHURI ; P. A. TIPTON.** *Plant Physiol,* 2002, vol. 130, 2061-2068
- **J. PESSOA et al.** *BMC Plant Biology,* 2010, vol. 20, 30
- **E. LUNDBERG et al.** *FEBS Journal,* 2009, vol. 276, 1999-2011
- **C. MATIOLLO et al.** *BBRC,* 2009, vol. 387, 712-716
- **J. B. FRENCH ; S. E. EALICK.** *Acta. Cryst.,* 2011, vol. D67, 671-677
- **S. HE et al.** *Appl. Environ. Microbiol.,* 2019, vol. 85 (19), e01107-19